# EUROPEAN PATENT APPLICATION

(11) **EP 4 640 830 A1**
(43) Date of publication of application: **29.10.2025**
(21) Application number: 24171984.8
(22) Date of filing: 23.04.2024
(51) Int. Cl.: C12N 9/22, C12N 15/11, C12N 15/90

(54) **MEANS AND METHODS FOR SAFE AND EFFICIENT GENE EDITING IN CELLS**

(71) Applicant: Fondazione Telethon ETS, 00185 Roma (IT); Ospedale San Raffaele S.r.l., 20132 Milano (MI) (IT)
(72) Inventor: Ferrari, Samuele, Milano (IT); Weber, Alessandra, Milano (IT); Naldini, Luigi, Milano (IT); Canarutto, Daniele, Milano (IT)
(74) Representative: Manfredi, Irene

(57) **Abstract**

The invention is directed to means and methods for efficient and safe genetic manipulation of a target cell, wherein a knock-out/knock in strategy is performed via HDR in a target gene that is haploinsufficient (and/or in single copy) in the cell.

## Description

### FIELD OF THE INVENTION

The present invention relates to means and methods for improving safety and efficiency of *ex vivo* or *in vitro* genetic manipulation of a cell.

### BACKGROUND

Gene therapy by integrating viral vectors have achieved excellent safety and efficacy results in clinical studies for the treatment of several inherited diseases, both hematological and non-hematological.

Yet, the use of semi-randomly integrating vectors still poses the risk of insertional mutagenesis, particularly when the viral vector bears highly active promoter/enhancer sequences¹⁵, and of non-physiological transgene expression.

Targeted integration of a therapeutic sequence in a pre-defined locus of interest may allow to overcome this limitation, and this can be achieved by gene editing (GE) through homology-directed repair (HDR), which relies on the use of artificial nucleases for precise modification of endogenous genes.

However, the application of GE by HDR, particularly in HSPCs, is currently constrained by low efficiency^{1,2} of GE and potential genotoxicity¹⁰⁻¹².

Low efficiency of GE is mainly due to the relative quiescent state of the most primitive and long-term repopulating HSPCs which makes them not very permissive to HDR, given the low proportion of cells in the S/G2 phases of the cell cycle at the time of editing. Despite significant improvements have been obtained with different strategies³⁻⁵, current editing efficiency remains suboptimal, achieving on average 10-20% of gene marking in long-term engrafting HSPCs ⁶⁻⁸. These levels of gene correction have been predicted to be safe and effective for the treatment of some diseases, such as in the case of X-linked Severe Combined Immunodeficiency (SCID-X1), in which the edited progeny would benefit of selective advantage over non-edited counterpart⁹. However, for other clinical applications, especially in conditions in which a higher threshold of edited cells is necessary for the correction of the pathological phenotype, it is crucial to improve the efficiency of gene editing as well as the yield of edited cells. This is even more relevant in gene editing of stem cells, such as HSPCs, for which it is essential to improve efficiency of gene editing in more primitive HSPCs.

GE per se can be a source of potentially genotoxic events in cells¹⁰⁻¹², both by gene disruption or HDR template integration at nuclease off-target sites and by loss of genetic material at the nuclease intended target site upon non-homologous (NHEJ) -mediated repair (e.g., long-range deletions and chromothripsis).

Selection strategies are promising methods to increase efficiency and safety at the same time: as for efficacy, by providing an increase in the proportion of the cells bearing the intended edit; as for safety, by purging out cells bearing imprecise and potentially genotoxic DNA repair outcomes, such as indels, large deletions and chromosomal translocations.

An approach for enrichment of HDR-edited HSPCs by "positive selection" (SMArT platforms): has been studied and validated by the present inventors (WO2020074729A1): this may be done by coupling the intended on-target editing outcome to transient expression of: a) cell-surface clinically compatible reporters that could allow for an ex vivo selection before transplantation; or b) biological selectors, i.e., molecules providing transient growth or engraftment advantage to the expressing cells, such as CXCR4, c-KIT, ITGA4.

Others have also developed an approach for enrichment of HDR-edited cells by "negative selection"¹³, providing growth, homing or engraftment disadvantage to cells not bearing the intended on-target editing outcome: in this case, the selection (called SLEEK, SeLection by Essential-gene Knock-out) was obtained by knocking out essential genes (*GAPDH*, *TBP and KIF11*) to be reconstituted in the HDR cassette together with the gene of interest. This approach has proven effective in iPSCs, B cells, NK cells, T cells, in which it was used with the purpose of increasing the yield of immune cells genetically modified to express anti-tumoral soluble mediators. Although the SLEEK platform increases the efficacy of the final cell product, it is not assuring safety, since cells bearing indels are not counter-selected. Indeed, in the SLEEK approach positively selected cells can bear one allele faithfully repaired by HDR while having on the other an unwanted and potentially genotoxic editing outcome (e.g., large deletions or chromosomal translocations), as confirmed also by the authors of the SLEEK approach¹³. Safety and preservation of genome integrity is however extremely important for gene therapy, especially for gene therapy involving HSPCs, since gene edited cells have to last life-long and potentially repopulate the haematopoietic system of the recipient, and may otherwise accumulate mutations triggering/promoting cell transformation.

The limitations of the prior art are overcome by the present invention, aimed at providing a negative selection strategy wherein cells carrying undesired gene editing outcomes at the target site (e.g. large deletions, translocations, duplications, indels) are spontaneously purged out (*in vitro* and mainly *in vivo*), with consequent indirect advantage to cells that are HDR-edited. Moreover, this invention allows for more uniform editing outcomes and characterization of the transcriptional profile of strong promoters integrated into the target locus.

### BRIEF DESCRIPTION OF THE INVENTION

The scope is achieved by the means and methods of the invention for efficient and safe genetic manipulation of a target cell, wherein a knock-out/knock in strategy is performed via HDR in a target gene that is haploinsufficient (and/or in single copy) in the cell.

In particular, the invention is directed to means and methods for *in vitro* or *ex vivo* engineering of cells (target cells), preferably of stem cells, more preferably of hematopoietic stem cells (HSCs) and/or of hematopoietic stem and progenitor cells (HSPCs), via targeted integration by HDR gene editing of an exogenous knock-in cassette for expressing a gene of interest in a target site that is located in a functional region of a gene that is haploinsufficient (and/or in single copy) in the target cell, wherein a functional region of the haploinsufficient gene, that is disrupted by gene editing cutting, is reconstituted upon integration of the knock-in cassette.

"Haploinsufficiency" in genetics describes a model of dominant gene action in diploid organisms, in which a single copy of the wild-type allele at a locus, even in heterozygous combination with a variant allele, is insufficient to produce the wild-type phenotype.

Often, knock-out by NHEJ of one of two alleles of a gene is not detrimental to the cell, as one copy of the gene is sufficient for the cell to survive and function properly.

Differently, in the case of haploinsufficient genes (i.e. genes for which two copies are required for proper function of the cell and/or genes that are present in single copy in the genome, such as X-linked genes in male somatic cells), their knockout or disruption by NHEJ results detrimental to the cell.

In the latter scenario, NHEJ in HSPCs may lead to drastic impairments in the survival, engraftment, homing, and/or self-renewing of cells, which may also eventually die.

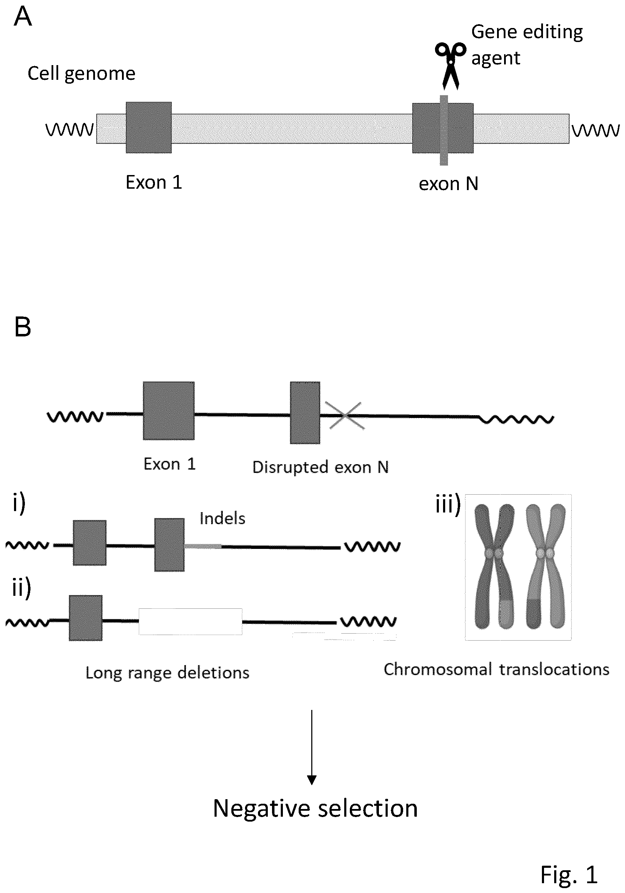

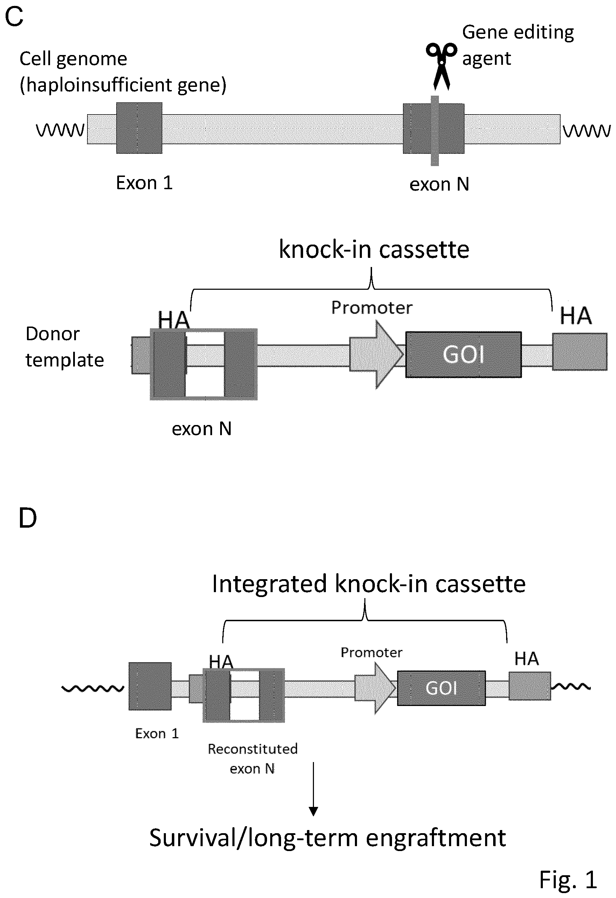

Therefore, among the target cells undergoing gene editing in a haploinsufficient gene, according to the invention, those bearing biallelic or monoallelic knockout of the target genes, including those carrying aberrant repair events at the target site, such as long-range deletions or chromothripsis, are spontaneously purged out and counter-selected (Fig. 1 A and B). Conversely, those bearing biallelic HDR-mediated integration (or monoallelic in the case of X-linked genes in males) of a template reconstituting the target gene open reading frame, simultaneously carrying the knock-in cassette for expressing the gene of interest, preserve their biological functions and are enriched (Fig. 1 C and D).

The present invention is then directed to a method for *in vitro* or *ex vivo* engineering of a cell, comprising introducing into a cell:
a) a gene editing agent comprising, or consisting of:
   a.i) a nuclease capable of introducing a double strand break (DSB) in a target site in the cell genome; and
   a.ii) a guide RNA (gRNA) targeting the target site;
   wherein the target site is located within a functional region of a target gene, wherein the target gene is a gene that is haploinsufficient and/or that is present in single copy in the cell;
      and
b) a donor template that comprises:
   b.i) a knock-in cassette comprising an exogenous polynucleotide encoding a gene product of interest, or a portion thereof; and
   b.ii) homology arms (HAs) on either side of the knock-in cassette, wherein the 5' homology arm (or "left" homology arm) has a sequence that is homologous (e.g. identical) to a sequence located 5' of the DSB in the target site of the cell genome and the 3' homology arm ("right" homology arm) has a sequence that is homologous to a sequence located 3' of the DSB in the target site of the cell genome;
   wherein the donor template comprises an exogenous polynucleotide having sequence capable of reconstituting the target gene upon integration of the knock-in cassette into the target site of the cell by homology-directed repair (HDR) of the DSB,
   such that integration of the knock-in cassette by HDR of the DSB results in a gene edited cell that expresses the gene product of the target gene and the gene product of interest.

With "reconstituting the target gene" it is meant reconstituting the functionality of the target gene. For instance, the polynucleotide reconstituting the target gene can reconstitute the open reading frame of the target gene and/or restore the function of a regulatory element of the target gene, such as a splicing site, or of any other element of the gene that regulates expression of the gene product, that was disrupted by the DSB.

Optionally, reconstitution of the target gene can include reconstitution of the target gene sequence; however, in accordance with the present invention, when exogenous sequences corresponding to endogenous sequences of the target gene are introduced by HDR in the DSB, said exogenous sequences are preferably codon-usage optimized sequences. Therefore, the gene product of the target gene that is reconstituted in the gene edited cell is encoded by a sequence that is homologous but not identical to the endogenous sequence.

According to the method of the invention, when the knock-in cassette is not integrated into the target site of the cell by homology-directed repair (HDR), in the correct position or orientation, the cell no longer expresses the gene product encoded by the target gene and it is therefore spontaneously purged out (*in vitro* and mainly *in vivo*), with consequent indirect advantage to cells that are HDR-edited.

The invention is also directed to a kit for *in vitro* or *ex vivo* engineering of a cell, comprising:
a) a gene editing agent comprising, or consisting of:
   a.i) a nuclease capable of introducing a double strand break (DSB) in a target site in the cell genome; and
   a.ii) a guide RNA (gRNA) targeting the target site;
   wherein the target site is located within a functional region of a target gene, wherein the target gene is a gene that is haploinsufficient and/or that is present in single copy in the cell;
      and
b) a donor template that comprises:
   b.i) a knock-in cassette comprising an exogenous polynucleotide encoding a gene product of interest, or a portion thereof; and
   b.ii) homology arms (HAs) on either side of the knock-in cassette, wherein the 5' homology arm (or "left" homology arm) has a sequence that is homologous (e.g. identical) to a sequence located 5' of the DSB in the target site of the cell genome and the 3' homology arm ("right" homology arm) has a sequence that is homologous to a sequence located 3' of the DSB in the target site of the cell genome;
   wherein the donor template comprises an exogenous polynucleotide having sequence capable of reconstituting the target gene, upon integration of the knock-in cassette into the target site of the cell by homology-directed repair (HDR) of the DSB,
   such that integration of the knock-in cassette by HDR of the DSB results in a gene edited cell that expresses the gene product of the target gene and the gene product of interest;
   the kit optionally comprising c) a cell population.

Further, the present invention is directed to a gene edited cell, obtainable by the method of the invention, and to pharmaceutical formulations comprising a population of the gene edited cells and suitable pharmaceutically acceptable excipients.

In a further aspect, the present invention is also directed to the kit or to the gene edited cell obtainable by the method of the invention, or the pharmaceutical formulation thereof, for use as a medicament, preferably for use in the treatment of a genetic disease, that can be treated by expressing the gene product of interest in a cell (gene therapy).

The present invention is then also directed to the kit or to the gene edited cell obtainable by the method of the invention, or the pharmaceutical formulation thereof, for use as medicament in gene therapy, preferably in *ex vivo* gene therapy.

The present invention is directed also to a method of treatment of a genetic disease comprising administering to a subject in need thereof a therapeutic amount of the kit, or gene edited cells of the invention, or pharmaceutical formulations thereof.

The invention is best understood from the following detailed description, when read in conjunction with the accompanying drawings.

### BRIEF DESCRIPTION OF FIGURES

Fig. 1 Schematic representation of the selection strategy according to the invention.
   **A.** schematic representation of a break (vertical line) operated by a gene editing agent in a target site of a functional region (exon N) of a target gene **B.** schematic representation of unwanted dysfunctional repair mechanisms, occurring after disruption of the target gene, such as (i) NHEJ (i), putting the disrupted exon N out of frame or producing genotoxic secondary byproducts with indels, (ii) long range deletions, and/or (iii) translocation, resulting fatal to the target cell and/or its progeny (negative selection). **C.** schematic representation of the elements of the kit for gene editing of the invention including a gene editing agent targeting a target site in exon N of a target gene and a donor template comprising a knock in cassette for integration by HDR in the target, expressing the disrupted exon N and a gene of interest (GOI) under a promoter, in between the homology arms (HA). **D.** schematic representation of the integration of the knock in cassette in the genome of the gene edited cell, resulting in repaired cell that is able to survive and engraft long-term.

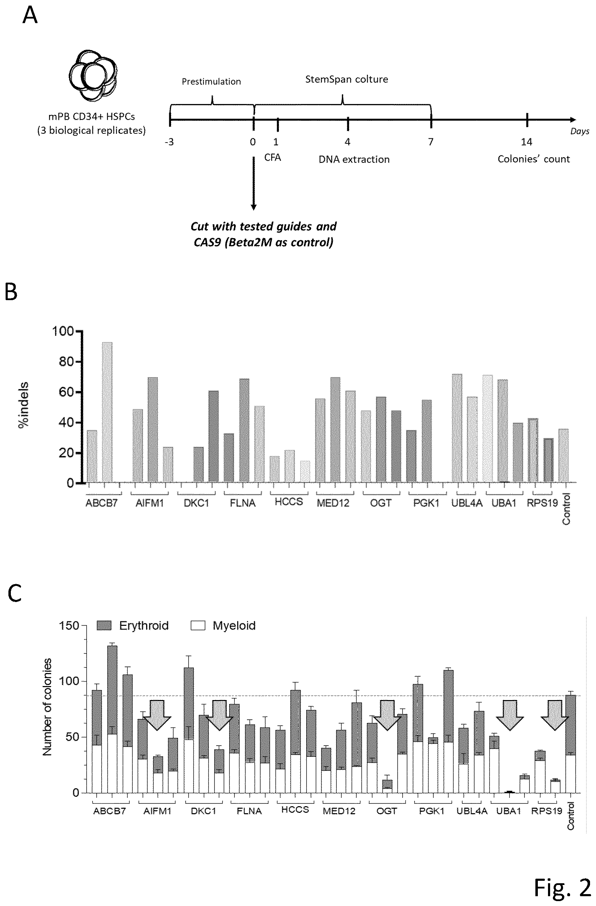

**Fig. 2****. Screening of candidate target genes *in vitro.***
   **A.** scheme of the experimental design of Example 1 for selecting preferred target genes in HSPCs **B.** Results of editing efficiency expressed as the percentage of indels present in the bulk culture of HSPCs, measured 4 days after editing, for each gene and each gRNA tested, represented in the graph by each bar, compared to control condition. **C.** Results of clonogenic assay expressed as the number of red and white colonies counted for each gene and each guide gRNA tested, represented in the graph by each bar, 14 days after editing. Absolute number of colonies are counted and compared to the control condition (dotted line). Results are shown as median values. The arrows point the guide RNAs that were most effective in impairing colonies *in vitro.*

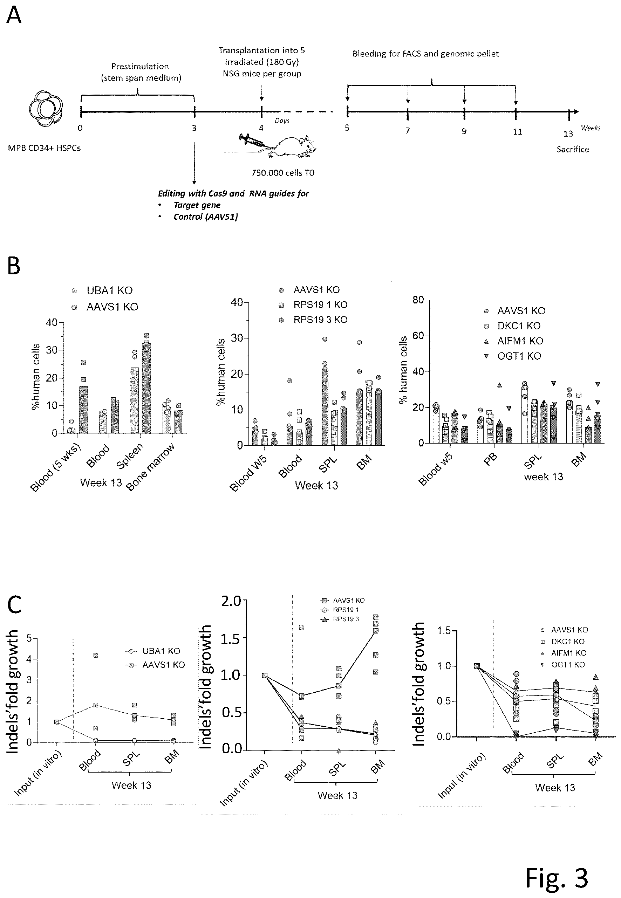

**Fig. 3****. *In vivo* engraftment and indels' purge out in xenograft models of edited HSPCs**
   **A.** scheme of the experimental design of Example 2 **B.** Human cells engraftment evaluated as percentage of cells staining positively for the surface antigen human CD45 at FACS. Results are shown for cells edited with gene editing agents knocking out (KO) *UBA1* gene (on the left), *RPS19* gene (in the middle) and *AIFM1, DKC1* and *OGT* genes (on the right). For *RPS19* two gRNAs were tested, gRNA 1 and 3, respectively disrupting exon 5 and 4 (RPS19 1 KO and RPS19 3 KO in the graph) **C.** Percentage of indels in the human graft, expressed in terms of fold growth, obtained when normalizing for *in vitro* editing efficiency where 1 is the fold growth at the moment of transplantation (input). Final graft fold is evaluated in blood, spleen (SPL) and bone marrow (BM) at week 13, for *UBA1* gene (on the left), *RPS19* gene (in the middle) and *OGT, DKC1* and *AIFMI* genes (on the right). Gene editing in genomic safe harbor *AAVS1* is taken as control.

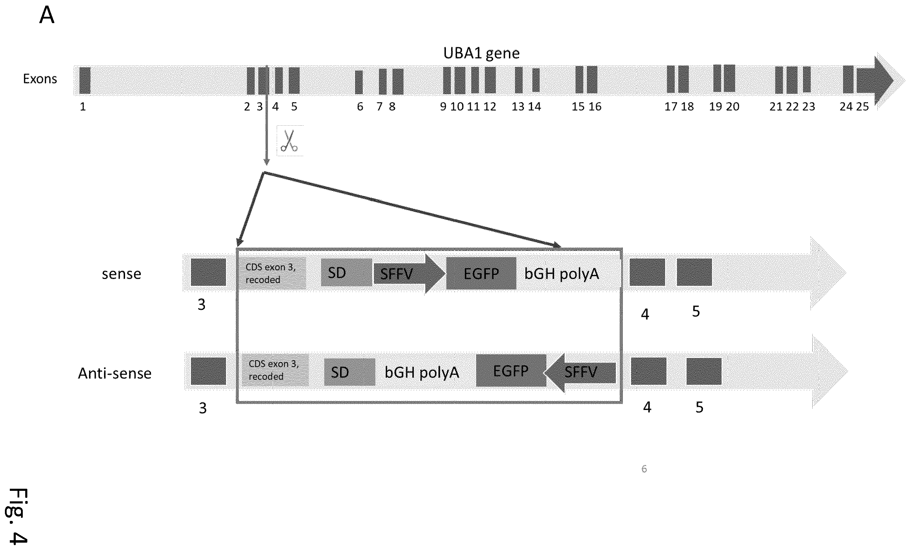

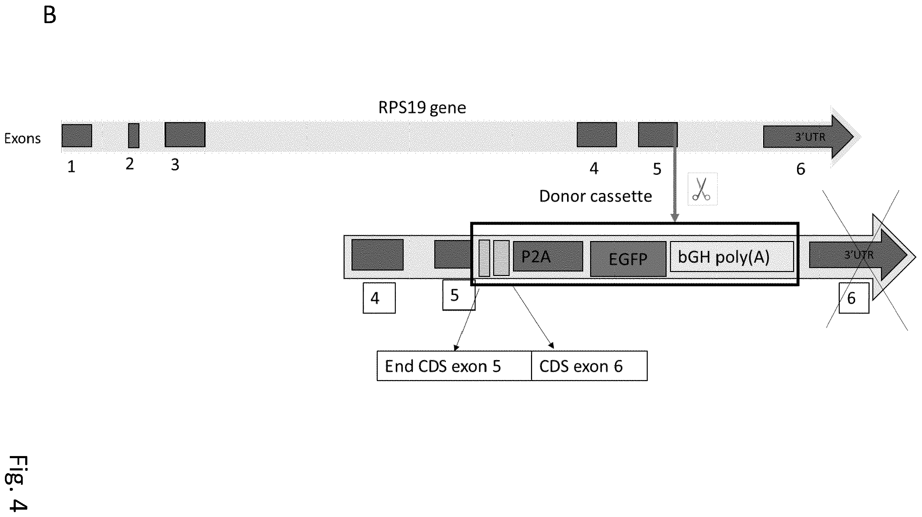

**Fig. 4****. HDR strategy in *UBA1* and *RPS19* loci.**
   **A.** scheme of the insertion in *UBA1* gene of the knock-in cassettes prepared in Example 3: in the upper part of the figure the native structure of UBA1 gene is shown, with exons (boxes numbered from 1 to 25), introns (light line) and 3' UTR region (right arrow); at the end of exon 3 a vertical arrow with scissors shows the target site that is cut by the gene editing agent, wherein a knock-in cassette is inserted as transgene by HDR. In the lower part of the figure it is shown the knock-in cassette inserted as transgene in the target site by HDR (homology arms are not shown); the knock-in cassette (sense) includes from the 5' end to the 3' end: the final portion of exon 3 of *UBA1* gene which is codon-usage optimized (recoded), a splicing donor domain (SD) and the cassette for expressing a gene of interest, consisting of a strong viral promoter (Spleen Focus-Forming Virus promoter, SFFV), enhanced green fluorescent protein (EGFP, mocking the gene of interest) and a bovine growth hormone (bGH) polyadenylation domain (polyA); an antisense cassette is also shown, wherein the cassette for expressing the gene of interest is in antisense direction as compared to the native gene. **B.** scheme of the insertion in *RPS19* gene of the knock-in cassettes cassette prepared in Example 3: in the upper part of the figure the native structure of *RPS19* gene is shown, with exons (boxes numbered from 1 to 6), introns (light line) and 3' UTR region (right arrow); at the end of exon 5 a vertical arrow with scissors shows the target site that is cut by the gene editing agent, wherein a knock-in cassette is inserted as transgene by HDR. In the lower part of the figure it is shown the knock-in cassette inserted in the target site by HDR (homology arms are not shown); the knock-in cassette includes from the 5' end to the 3' end: the final portion of the coding sequence (CDS) of exon 5, codon-usage optimized, and the CDS of exon 6, a P2A domain (encoding for self-cleaving peptide 2A) for ribosomal skipping in translation, and the cassette for expressing a gene of interest with EGFP (mocking the gene of interest), that is under the control of the endogenous promoter of the *RPS19* gene, followed by a bGH polyA domain.

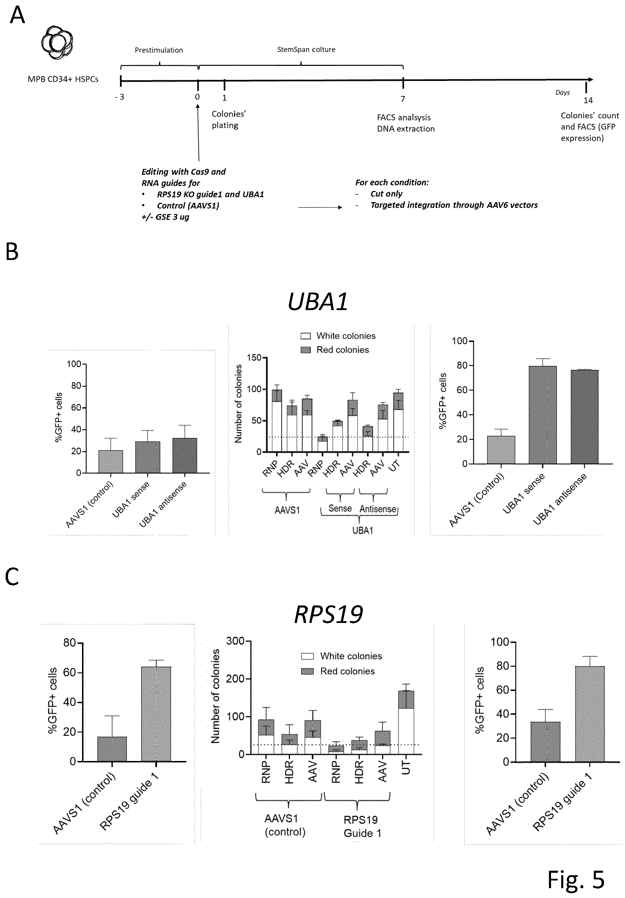

**Fig. 5****. clonogenic assay of HDR-edited HSPCs in *UBA1* and *RPS19* loci.**
   **A.** scheme of the experimental design of Example 4 for evaluating the efficacy of the method of the invention when targeting *UBA1* and *RPS19* loci. **B.** Results for *UBA1* locus: left graph shows the HDR efficiencies by FACS in the HDR-edited groups (with sense and anti-sense construct respectively); middle graph shows the absolute number of red and white colonies in each group; right graph shows the percentage of GFP positive cells among the colonies for HDR-edited groups. C. Results for *RPS19* locus: left graph shows the HDR efficiencies by FACS in the HDR-edited groups (with sense and anti-sense construct respectively); middle graph shows the absolute number of red and white colonies in each group (RNP= KO only condition, AAV=AAV vector without RNP delivery, HDR=full HDR condition, UT=untreated; right graph shows the percentage of GFP positive cells among the colonies for HDR-edited groups.

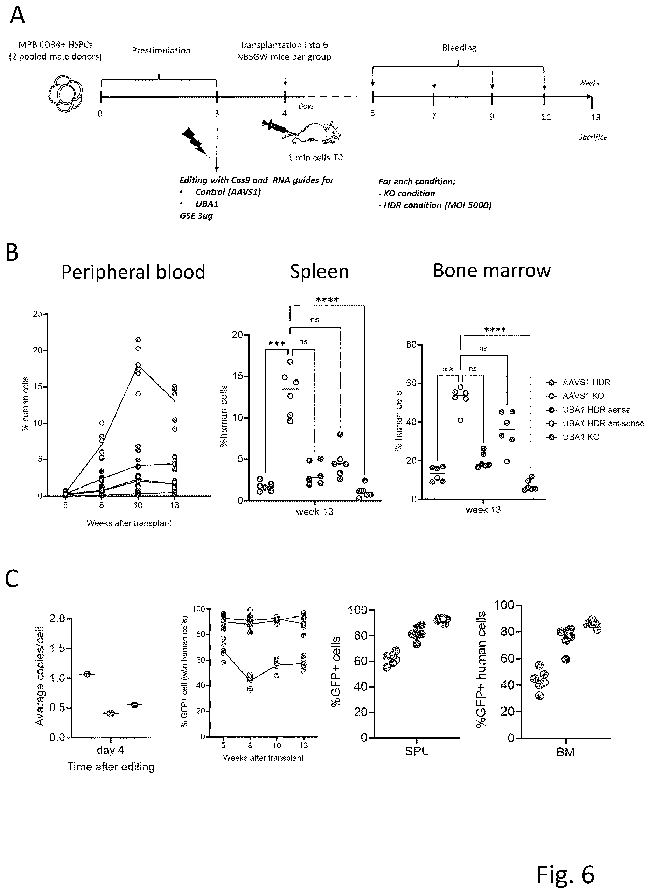

**Fig. 6****. in vivo engraftment of HDR-edited HSPCs in *UBA1* locus.**
   **A.** scheme of the experimental design of Example 5 for evaluating the efficiency of *in vivo* engraftment of HDR-edited HSPCs in *UBA1* locus. For each group of treatment (*AAVS1*, *UBA1* sense and antisense constructs) there was an RNP only group (KO) and a full HDR group. 30 NBSGW female mice (6 per group) were used, without need for irradiation prior to transplantation. **B.** human engraftment at sacrifice (13 weeks after transplantation) measured as percentage of hCD45 positive cells in mice's peripheral blood (left), spleen (middle) and bone marrow (right). **C.** HDR efficiency, measured as percentage of edited alleles (*in vitro*, figure on the left) evaluated 4 days after editing as average edited copies per cell in each HDR-edited condition, and as percentage of GFP expression (*in vivo*, figure on the right) within human cells, respectively, from left to right, in the peripheral blood, spleen and bone marrow, 13 weeks after transplantation.

### DETAILED DESCRIPTION OF THE INVENTION

Unless defined otherwise, all technical and scientific terms used herein have the same meaning as commonly understood by one of ordinary skill in the art to which this invention belongs.

It must be noted that as used herein and in the appended claims, the singular forms "a", "an", and "the" include plural referents unless the context clearly dictates otherwise. Thus, for example, reference to "a cell" includes a plurality of cells, such as a population of cells.

The term "about" or "approximately" in relation to a numerical means, a range of values that fall within 10% greater than or less than the value. For example, about x means x± (10% * x).

The terms "engineered" or "genetically modified" or "genetically manipulated", as referred e.g. to cells, are herein used interchangeably.

"Engineering" or "genetic modification" or "genetic manipulation" include "gene transfer", "gene editing" and "epigenetic editing". In the present invention, the terms "engineering" or "genetic modification", referred to a cell or organism that is "engineered" or "genetically modified" mean "gene editing".

The term "gene transfer" " or "gene delivery" refers to the transfer of genetic material (e.g., DNA or RNA) of interest into a cell, typically to treat or prevent a genetic or acquired disease or condition. For instance, the term "gene transfer" " or "gene delivery" can refer to the addition of a copy of a gene into the genome of a cell, such as a correct copy of a gene that is completely or partially deleted, or completely or partially not functional, in a cell (gene therapy). In particular, the genetic material of interest typically encodes a product (e.g., a protein polypeptide, peptide or functional RNA) whose production *in vivo* is desired. For example, a genetic material of interest can encode a gene product that is an enzyme, hormone, receptor, or polypeptide of therapeutic value. "Gene transfer" or "gene delivery" also refer to methods or systems for reliably introducing DNA or RNA of interest into a host cell. Such methods can result in transient expression of non-integrated transferred DNA, extrachromosomal replication and expression of transferred replicons (e.g., episomes), or integration of transferred genetic material into the genomic DNA of host cells.

The term "gene editing" refers to the modification of the genome of a cell at a specific location to correct or alter a genetic sequence. The term "gene editing" refers in particular to a type of genetic engineering in which a nucleic acid is inserted, deleted or replaced in a cell. In accordance with the present invention, the term "gene editing" encompasses targeted disruption of a gene coding sequence and precise sequence substitution for *in situ* and targeted transgene insertion into a predetermined locus in the genome of a cell.

A "gene transfer agent" is an agent capable of transferring a gene into a cell, such as a transgene and/or a vector for delivering a transgene, such as a vector comprising a transgene.

The term "gene editing agent" or "gene editor" refers to an agent capable of editing a target site in the genome of a cell, e.g. by mediating disruption of the target site and/or correcting the target site. In accordance with the present invention, a gene editing agent is preferably an agent capable of disrupting a target site in the genome of a cell. The term "vector" refers to a particle capable of delivering, and optionally expressing, one or more polynucleotides of interest into a host cell. Examples of vectors include, but are not limited to, naked DNA or RNA expression vectors, plasmid, cosmid or phage vectors, DNA or RNA expression vectors associated with cationic condensing agents, DNA or RNA expression vectors encapsulated in liposomes, and certain eukaryotic cells, such as producer cells. The vector can be a cloning vector, suitable for propagation and for obtaining polynucleotides, gene constructs or expression vectors incorporated to several heterologous organisms. A vector is capable of transferring nucleic acid sequences to target cells, therefore also viral vectors, non-viral vectors, particulate carriers, and liposomes are included in the term "vector". Typically, "vector construct", "expression vector" and "gene transfer vector" mean any nucleic acid construct capable of directing the expression of a nucleic acid of interest and which can transfer nucleic acid sequences to target cells. Thus, the term includes cloning and expression vehicles, as well as viral vectors.

The term "recombinant plasmid" or "plasmid" refers to a small, circular, double- stranded, self-replicating DNA molecule obtained through genetic engineering techniques capable of transferring genetic material of interest to a cell, which results in production of the product encoded by that said genetic material (e.g., a protein polypeptide, peptide or functional RNA) in the target cell. Furthermore, the term "recombinant plasmid" or "plasmid" also refers to a small, circular, double-stranded, self-replicating DNA molecule obtained through genetic engineering techniques used during the manufacturing of viral vectors as carriers of the recombinant vector genome.

The term "recombinant viral vector" or "viral vector" refers to an agent obtained from a naturally-occurring virus through genetic engineering techniques capable of transferring genetic material (e.g., DNA or RNA) of interest to a cell, which results in production of the product encoded by that said genetic material (e.g., a protein polypeptide, peptide or functional RNA) in the target cell. Herein, the terms "vector transgene" or "recombinant vector transgene" refer to a transgene that is transferred to the recipient cell upon transduction. The term "viral vector" or "recombinant viral vector", as used herein, also refers to the recombinant viral particles being a packaged viral vector, capable of binding to and entering recipient cells, delivering the vector transgene.

Viral delivery include but is not limited to delivery by adenoviral vectors, adeno-associated viral (AAV) vectors, herpes viral vectors, retroviral vectors, lentiviral vectors, integrase-defective lentiviral vectors and baculoviral vectors.

The terms "nucleotide sequence" or "isolated nucleotide sequence" or "polynucleotide sequence" or "polynucleotide" or "isolated polynucleotide sequence" are interchangeably used herein and refer to a nucleic acid molecule, either DNA or RNA, containing deoxyribonucleotides or ribonucleotides respectively. The nucleic acid may be double stranded, single stranded, or contain portions of both double stranded or single stranded sequence.

The terms "variant" refers to biologically active derivatives of the reference molecule that retain desired activity. In general, the term "variant" refers to molecules having a native sequence and structure with one or more additions, substitutions (generally conservative in nature) and/or deletions, relative to the native molecule, so long as the modifications do not destroy biological activity, and which are "substantially homologous" to the reference molecule. In general, the sequences of such variants will have a high degree of sequence homology to the reference sequence, e.g., sequence homology of more than 50%, generally more than 60-70%, even more particularly 80-85% or more, such as at least 90-95% or more, when the two sequences are aligned. In accordance with the present invention, a variant of any biomolecule is a biomolecule that has a nucleic acid or aminoacidic sequence having a % of identity of 50%, 60%, 70%, 80%, 90%, 95%, or 99% to the wild-type nucleic acid or aminoacidic sequence and that retains the biological activity of the wild-type biomolecule. In preferred aspects, the term "variant" of a polynucleotide sequence is used herein to indicate serum-free sequence having a % of identity of at least 90%, 95% or 99% to said polynucleotide sequence. In preferred aspects, the term "variant" of a polynucleotide sequence is used herein to indicate a sequence that is a codon-optimized sequence for expressing the biomolecule encoded by said sequence.

The terms "% sequence identity", "% identity" or "% sequence homology" refer to the percentage of nucleotides or amino acids of a candidate sequence that are identical to the nucleotides or amino acids in the sequence of reference, after aligning the sequences to achieve the maximum % sequence identity. In a preferred embodiment, sequence identity is calculated based on the full length of two given sequences or on part thereof. The % sequence identity can be determined by any methods or algorithms established in the art, such as the ALIGN, BLAST and BLAST 2.0 algorithms and followings. Herein, the "% sequence identity", "% identity" "or "% sequence homology" is calculated dividing the number of nucleotides or amino acids that are identical after aligning the sequence of reference and the candidate sequence, by the total number of nucleotides or amino acids in the sequence of reference and multiplying the result by 100. In accordance with degeneration of genetic code, variants include sequences where at least one base of the base sequence of a gene is replaced with a different type of base, without changing the amino acid sequence of the polypeptide expressed from the gene. Variants also include codon-optimized sequences and sequences comprising mutated or added nucleotides, e.g., for cloning needs. Codon optimization has previously been described in WO 1999/41397 and WO 2001/79518. Different cells differ in their usage of particular codons. This codon bias corresponds to a bias in the relative abundance of particular tRNAs in the cell type. By altering the codons in the sequence so that they are tailored to match with the relative abundance of corresponding tRNAs, it is possible to increase expression. By the same token, it is possible to decrease expression by deliberately choosing codons for which the corresponding tRNAs are known to be rare in the particular cell type. Thus, an additional degree of translational control is available. Codon usage tables are known in the art for mammalian cells, as well as for a variety of other organisms. Variants also include sequences encoding fragments of any biomolecule, i.e., a shorter form of the biomolecule, such as a truncated form, that retains the biological activity of the wild-type biomolecule.

The terms "codify", "coding" or "encoding" refer to the genetic code that determines how a nucleotide sequence is translated into a polypeptide or a protein. The order of the nucleotides in a sequence determines the order of amino acids along a polypeptide or a protein.

The term "transcriptional regulatory region" or "regulatory element, or region", as used herein, refers to a nucleic acid fragment capable of regulating the expression of one or more genes. The regulatory regions of a polynucleotides of the invention may include a promoter, plus response elements, activator and enhancer sequences for binding of transcription factors to aid RNA polymerase binding and promote expression, and operator or silencer sequences to which repressor proteins bind to block RNA polymerase attachment and prevent expression.

The term "promoter" must be understood as a nucleic acid fragment that functions to control the transcription of one or more polynucleotides e.g. coding sequences, which is placed 5' upstream of the polynucleotide sequence(s), and which is structurally identified by the presence of a binding site for DNA dependent RNA polymerase, transcription initiation sites and, but not limited to, binding sites for transcription factors, repressors, and any other nucleotide sequences known in the art to act directly or indirectly to regulate the amount of transcription from the promoter. A promoter is said to be operatively linked to a nucleotide sequence or to drive the expression of it when it can initiate transcription of said nucleotide sequence in an expression system using a gene construct comprising said promoter operably linked to a nucleotide sequence of interest using a suitable assay such a RT- qPCR or Northern blotting (detection of the transcript). The activity of said promoter may also be assessed at the protein level using a suitable assay for the encoded protein such as Western blotting or an ELISA. A promoter is said to be capable to initiate transcription if a transcript can be detected or if an increase in a transcript or protein level is found of at least 5%, 10%, 15%, 20%, 30%, 40%, 50%, 60%, 70%, 80%, 90%, 100%, 200%, 300%, 500%, 1000%, 1500% or 2000% as compared to transcription using a construct which only differs in that it is free of said promoter.

The term "constitutive" promoter refers to a promoter that is active under most physiological and developmental conditions. An "inducible" promoter is a promoter that is preferably regulated depending on physiological or developmental conditions. A "tissue-specific" promoter is preferably active in specific types of cells/tissues. A "ubiquitous" promoter may be defined as a promoter that is active in many or in any different tissue(s).

A "strong promoter" is generally meant to indicate a promoter capable of inducing expression of high levels of a gene product under its control.

"Host cells," "cells", "cell lines," "cell cultures", "engineered cells" and other such terms denoting microorganisms or higher eukaryotic cell lines cultured as unicellular entities refer to cells which can be, or have been, used as recipients for gene modification include the original progeny of the original cell.

The term "primary cell" means a cell isolated from an organism, e.g., a mammal, which is grown in tissue culture (i.e., in vitro) for the first time before subdivision and transfer to a subculture. Primary cells and stem cells, can be modified through introduction of one or more polynucleotides, polypeptide, and/or prime editing compositions (e.g., through transfection, transduction, electroporation, and the like) and further passaged.

The terms "culture or culturing", "growth or growing", referred to cells, are used herein interchangeably and are meant to indicate maintenance of a cell population *in vitro* or ex vivo, preferably including expansion of the cell population.

The term "incubating" or "incubation", as referred herein to cells and agents according to the invention, means contacting a cell with an agent, e.g., a means for delivering a gene editing agent, and maintaining the contact for a time suitable for delivering the agent into the cell. The term "engraftment" as used herein refers to the ability of cells to populate and survive in a subject following their transplantation, i.e., in the short and/or long term after transplantation. For example, engraftment may refer to the number and/or percentages of hematopoietic cells descended from transplanted hematopoietic stem and/or progenitor cells (e.g., graft-derived cells) that are detected about 1 day to 24 weeks, 1 day to 10 weeks, or 1-30 days or 10-30 days after transplantation. In a xenograft model of human hematopoietic stem and/or progenitor cell engraftment and repopulation, engraftment may be evaluated in the peripheral blood as the percentage of cells deriving from the human xenograft (e.g., positive for the CD45 surface marker), for example. Engraftment may be readily analyzed by the skilled person. For example, transplanted hematopoietic stem and/or progenitor cells may be engineered to comprise a marker (e.g., a reporter protein, such as a fluorescent protein), which can be used to quantify the graft-derived cells. Samples for analysis may be extracted from relevant tissues and analyzed ex vivo (e.g., using flow cytometry).

The terms "treatment", "treating", "treat" and the like, as used herein, refer to the administration of a compound, agent, composition or formulation of the invention to obtain a desired pharmacologic and/or physiologic effect. The effect can be prophylactic in terms of completely or partially preventing a disease or symptom(s) thereof and/or may be therapeutic in terms of a partial or complete stabilization or cure for a disease and/or adverse effect attributable to the disease or control of disease progression. The terms "prevent," "preventing," and "prevention", as used herein, refer to inhibiting the inception or decreasing the occurrence of a disease in a subject. Prevention may be complete (e.g., the total absence of pathological cells in a subject) or partial. Prevention also refers to a reduced susceptibility to a clinical condition. Control of disease progression is understood as the achievement of the beneficial or desired clinical results that include, but are not limited to, reduction of the symptoms, reduction of the duration of the disease, stabilization of pathological states (specifically to avoid additional deterioration), delay of the progression of the disease, improvement in the pathological state, and remission (both partial and total). The control of progression of the disease also involves an extension of survival, compared with the expected survival if treatment is not applied.

In particular, in accordance with the present invention, the terms "treatment", "treating", "treat" and the like, as used herein, preferably refer to the administration of a compound, composition or formulation to cure, prevent, delay and/or control the clinical manifestations of a pathology. The term "effective amount" or "therapeutical amount" refers to a quantity of a composition, for example a gene editing agent(s) and/or a gene therapy composition, that can be sufficient to result in a desired activity upon introduction into a subject as disclosed herein. An effective amount of a gene editing compositions and/or gene therapy composition can be provided to the target gene or cell. In some embodiments, the "effective amount" or "therapeutically effective amount" is the amount of a composition that is required to ameliorate the symptoms of a disease relative to an untreated patient. In some embodiments, an effective amount is the amount of a composition sufficient to introduce an alteration in a gene of interest in a cell (e.g., a cell *in vitro*, *ex vivo* or *in vivo*). In some embodiments, an effective amount can be an amount to induce, when administered to a population of cells, a certain percentage of the population of cells to have a correction of a mutation or to bear a transgene. For example, in some embodiments, an effective amount can be the amount to induce, when administered to or introduced to a population of cells, installation of one or more intended nucleotide edits in the target gene. The term "individual" or "subject" herein refers to a mammal, preferably human or non-human mammal, more preferably a human, or a mouse, rat, other rodents, rabbit, dog, cat, pig, cow, horse or primate. Those in need of treatment include those already inflicted as well as those in which prevention is desired (e.g., those with no symptoms but diagnosed with the genetic disorder, etc.).

In a first aspect, the present invention is directed to a method for *in vitro* or *ex vivo* engineering of a cell, comprising introducing into a cell:
a) a gene editing agent comprising, or consisting of:
   a.i) a nuclease capable of introducing a double strand break (DSB) in a target site in the cell genome; and
   a.ii) a guide RNA (gRNA) targeting the target site;

   wherein the target site is located within a functional region of a target gene, wherein the target gene is a gene that is haploinsufficient and/or that is present in single copy in the cell;
   and
b) a donor template that comprises:
   b.i) a knock-in cassette comprising an exogenous polynucleotide encoding a gene product of interest, or a portion thereof; and
   b.ii) homology arms (HAs) on either side of the knock-in cassette, wherein the 5' homology arm (or "left" homology arm) has a sequence that is homologous (e.g. identical) to a sequence located 5' of the DSB in the target site of the cell genome and the 3' homology arm ("right" homology arm) has a sequence that is homologous to a sequence located 3' of the DSB in the target site of the cell genome;

   wherein the donor template comprises an exogenous polynucleotide having sequence capable of reconstituting the target gene upon integration of the knock-in cassette into the target site of the cell by homology-directed repair (HDR) of the DSB,
   such that integration of the knock-in cassette by HDR of the DSB results in a gene edited cell that expresses the gene product of the target gene and the gene product of interest.

The invention is also directed to a kit for *in vitro* or *ex vivo* engineering of a cell, comprising:
a) the gene editing agent, and
b) the donor template,
as described above; optionally comprising also:
c) a cell population.

According to preferred embodiments of the present invention, the cell is a mammalian cell, more preferably, the cell is a human cell. Optionally the cell is a male cell (i.e. a cell having one X chromosome and one Y chromosome).

Preferably, the cells is a myeloid progenitor cell.

In particularly preferred embodiments of the invention, the cell is stem cell, more preferably a hematopoietic stem cells (HSCs) or a hematopoietic stem and progenitor cells (HSPCs). Most preferably, said cell is a CD34+ HSPC.

The cell can be a mobilized peripheral blood cell (mPB cell), a cord blood cell (CB cells), or a bone marrow cell (BM cell).In preferred embodiments, the cell is provided after *in vitro* or *ex vivo* selection and/or expansion of a population of cells; more preferably the cell is provided after selection and/or expansion of a specific population of cells, such as a population of CD34+ HSPCs.

Therefore, preferably, the method of the invention comprises a step of selecting and expanding *ex vivo* a population of cells isolated from a subject, prior to introducing into said cell the gene editing agent.

More preferably, the method of the invention comprises as a step of selecting and expanding *ex vivo* CD34+ HSPCs obtained and isolated by leukapheresis or bone marrow harvest, prior to incubating said cells with at least one gene editing agent.

Optionally the cell that is genetically modified in the method of the invention is an induced pluripotent stem cell (iPSC).

In some embodiments the cell is a T cell, a B cell or a NK cell.

According to the present invention, suitable target genes are genes that are haploinsufficient in the cell type to be engineered, optionally genes that are present in a single copy in the cell to be engineered.

Clearly, suitable target genes include X-linked genes that are present in single copy in male cells, being thus by definition haploinsufficient.

In preferred embodiments, the target gene is one of the genes listed in Table 1 that follows.

**Table 1**

| **Gene** | **Ensemble ID** | **Location** | **Description** | **Evidence/mechanism for haploinsufficiency in HSPCs** |
|---|---|---|---|---|
| RPS19 | ENSG00000105372 | 19q13.2 | Ribosomal Protein S19 | Mutated in bone marrow failure associated disease (Blackfan Diamond anemia) |
| ABCB7 | ENSG00000131269 | Xq13.3 | ATP Binding Cassette subfamily B member 7 | Essential for hemopoiesis (transport of heme) |
| AIFM1 | ENSG00000156709 | Xq26.1 | Apoptosis Inducing Factor Mitochondria Associated 1 | Lethal in mice embryos; ablation of AIFM1 early during hematopoiesis causes hematopoietic stem cell (HSC) loss, thymopoiesis blockade, and delayed development of the T-cell, B-cell, and erythroid lineages in mouse model |
| DKC1 | ENSG00000130826 | Xq28 | Dyskerin Pseudouridine Synthase 1 | Mutation associated to bone marrow failure syndrome (Dyskeratosis Congenita) |
| FLNA | ENSG00000196924 | Xq28 | Filamin A | Mutation is pre- or peri-natal phase is deadly in male |
| HCCS | ENSG00000004961 | Xp22.2 | Holocytochrome C Synthase | Mutation lethal in utero in male |
| MED12 | ENSG00000184634 | Xq13.1 | Mediator Complex Subunit 12 | Involved in initiation of transcription |
| OGT | ENSG00000147162 | Xq13.1 | O-linked N-acetylglucosami ne (GlcNAc) Transferase | Lethal in mouse embryonic stem cells and essential for HSPCs maintenance. OGT disruption in HSCs leads to rapid loss of HSPCs with increased ROS and apoptosis. |
| PGK1 | ENSG00000102144 | Xq21.1 | Phosphoglycerat e kinase 1 | Conversion of 1,3-diphosphoglycerate to 3-phosphoglycerate |
| UBL4A | ENSG00000102178 | Xq28 | Ubiquitin Like 4A | "Housekeeping gene": higher rate of neonatal death in KO mice |
| UBA1 | ENSG00000130985 | Xp11.23 | Ubiquitin Like modifier Activating Enzyme 1 | Acquired mutations are linked to clonal hematopoiesis (VEXAS syndrome) |

Preferably, the target gene is selected from: *UBA1, RPS19, AIFM1, DKC1,* and *OGT.* More preferably, the target gene is *UBA1, RPS19, or OGT.*

Further genes can be targeted as well in the method of the invention, as long as said genes are haploinsufficient in the target cell.

In preferred embodiments of the present invention, the cell is HSPC and the target gene is selected from: *UBA1, RPS19, ABCB7, AIFM1, DKC1, FLNA, HCCS, MED12, OGT, PGK1,* and *UBL4A,* more preferably from *UBA1, RPS19, AIFM1, DKC1,* and *OGT,* most preferably, the target gene is *UBA1, RPS19,* or *OGT.*

In particularly preferred embodiments, the target gene is *RPS19* and the cell is a HSPC.

A functional region of a target gene is a region that is essential for the functionality of the gene and/or for the expression of the gene product; functional regions include regulatory regions of the target gene and coding regions of the target gene.

In preferred embodiments, the target site is located within a coding region of the target gene, such as an exon of the target gene, and the polynucleotide having sequence capable of reconstituting the open reading frame of the target gene upon integration of the knock-in cassette by HDR.

The gRNA of the gene editing agent of the method of the invention is preferably selected within the list of gRNAs of Table 2, targeting a target site having sequence ("target sequence") comprising, or consisting of, any one of SEQ ID NO: 1 to 33.

Typically, in the context of gene editing, the "target sequence" is a sequence to which a guide sequence is designed to have complementarity, where hybridization between the target sequence and a guide sequence promotes the formation of a complex comprising the nuclease. The target sequence may comprise any polynucleotide, such as DNA or RNA polynucleotides. In some embodiments, the target sequence is located in the nucleus or cytoplasm of the cell. In some embodiments, the target sequence may be within an organelle of the cell.

**Table 2**

| **SEQ ID NO:** | **Target sequence** | **Guide RNA ID** | **Targeted region on the target gene** |
|---|---|---|---|
| 1 | AAACGCAGGCCTTACCTGTC | RPS19_1 | Exon 5 |
| 2 | GTCAGTTTGCGGCCGCTGTG | RPS19_2 | Exon 5 |
| 3 | TACCCCCAGCTTCCACAGCG | RPS19_3 | Exon 4 |
| 4 | TTGTTGGCAGCTGCCACCTG | RPS19_4 | Exon 6 |
| 5 | TAGACGAGGGCCTTTACTCC | UBA1 ex3 #2 | Exon 3 |
| 6 | AGTGAAGCAGACATAGACGA | UBA1 ex3 #3 | Exon 3 |
| 7 | ACGCGACGTTTCTTGGACAG | UBA1 ex2 #4 | Exon 4 |
| 8 | TCAGCATGTTTCCCGACATC | ABCB7_A | Exon 5 |
| 9 | CACCTTGTTTACTCTACTCA | ABCB7_B | Exon 9 |
| 10 | AACAAAGCAGATAATGATGC | ABCB7_C | Exon 7 |
| 11 | AATGCTATTGTGCAATCCGT | AIFM_D | Exon 11 |
| 12 | AATAGCTTCATAGCCAACAT | AIFM_E | Exon 14 |
| 13 | CTAGAGGAACATGCCATCGC | AIFM_F | Exon 2 |
| 14 | AAGTTGCTAAGTTGGACACG | DKC1_G | Exon 3 |
| 15 | GGATTTGAACCACATGCAAG | DKC1_H | Exon 4 |
| 16 | AGGGCCTTCTGGACAAGCAT | DKC1_I | Exon 12 |
| 17 | GCCCGTTACCAATGCGCGAG | FLNA_J | Exon 4 |
| 18 | CTGCCAGGCATCGAGCCCAC | FLNA_K | Intron 5 and exon 6 |
| 19 | CGAGGTGACGGGGACTCATA | FLNA_L | Exon 7 |
| 20 | TCACAATCAGAATAACGAGC | HCCS_M | Exon 5 |
| 21 | GAATAACGAGCAGGCTTGGA | HCCS_N | Exon 5 |
| 22 | GGATGAGGATATCAGTCAGA | HCCS_O | Exon 5 |
| 23 | AAGGCCGTCAGTTCATCCTG | MED12_P | Intron 1 and exon 2 |
| 24 | CTCAGAGATTGCTGCATAGT | MED12_Q | Exon 4 |
| 25 | CGTCAGCTTCAATCCTGCCA | MED12_R | Exon 2 |
| 26 | TCCCTGTTCTCGTTTGATAT | OGT_S | Exon 8 |
| 27 | GGATATAGCATACTATGATG | OGT_T | Exon 12 |
| 28 | GCTCAAAGCCCTGGGTCGCT | OGT_U | Exon 4 |
| 29 | CCAGCTCATCAATAATATGC | PGK_V | Exon 7 |
| 30 | GCTCATAAGGACTACCGACT | PGK_W | Exon 3 |
| 31 | GCACACCATCAGGCCGGCCT | PGK_X | Exon 3 |
| 32 | CGACTCTCGGATTATAGCAT | UBLA4_Y | Exon 3 |
| 33 | GTTCGATGTCGTCCAGCGTC | UBLA4_Z | Exon 4 |

More preferably, the gRNA of the gene editing agent of the method of the invention is a gRNAs targeting a target site having sequence comprising, or consisting of SEQ ID NO: 1, 3, 5, 12, 16, or 27.

Said gRNAs show very high cutting efficiency. In this way, the amount of residual non-edited cells(that are not purged out, not bearing any kind of detrimental indels on the target gene) is very low or absent.

The gRNA of the gene editing agent targets the nuclease to the target site in the genome of the cell (van der Oost et al. (2014) Nat. Rev. Microbiol. 12: 479-92).

Preferred nucleases suitable for gene editing of a target gene according to the present invention include zinc finger nucleases (ZFNs), transcription activator like effector nucleases (TALENs), and the clustered regularly interspaced short palindromic repeats (CRISPR)/Cas nuclease (Gaj, T. et al. (2013) Trends Biotechnol. 31: 397-405). Meganucleases (Silve, G. et al. (2011) Cur. Gene Ther. 11: 11-27).

According to particularly preferred aspects of the invention, the gene editing agent introduced in the cell in the method of the invention comprises a nuclease being selected from: zinc-finger nuclease, a transcription activator like effector nuclease (TALENs), and a Cas nuclease. According to particularly preferred embodiments of the invention, the gene editing agent is comprises a gRNA, which sequence-specifically binds to the target site, and a Cas nuclease (e.gCas9 or Cas12a), capable of introducing a double strand break (DSB) in the target site.

The nuclease can be introduced in the cell as DNA encoding the nuclease, or as RNA transcript thereof.

In some embodiments, the gene editing agent is introduced in the cell by a viral vector comprising a polynucleotide encoding the gene editing agent.

Therefore, the method of the invention preferably comprises a step of transducing the cell with a viral vector comprising a polynucleotide encoding the gene editing agent.

Viral vectors suitable for delivering the at least one gene editing agent include, but are not limited to, adenoviral vectors, adeno-associated viral (AAV) vectors, herpes viral vectors, retroviral vectors, lentiviral vectors and baculoviral vectors. More preferred viral vectors are lentiviral vectors (LVs), or integration-defective lentiviral vectors (IDLVs).

In some embodiments, the gene editing agent is delivered in the cell by a non-viral vector.

Non-viral delivery systems include, but are not limited to, DNA transfection methods. Typical transfection methods include electroporation, DNA biolistics, lipid-mediated transfection, compacted DNA-mediated transfection, liposomes, immunoliposomes, lipofectin, cationic agent-mediated transfection, cationic facial amphiphiles (CFAs) (Nature Biotechnology (1996) 14: 556), nanoparticles, and combinations thereof.

Any RNA of the gene editing agent, such as the gRNA or a nuclease-encoding mRNA, can be delivered in the form of RNA. The RNA can directly contact the target gene or can be introduced into a cell using any suitable technique for introducing nucleic acids into cells (e.g., microinjection, electroporation, transfection).

An exogenous polynucleotide template is introduced in the cell for recombination into the targeted locus comprising the target site. This is referred to as "donor template".

Preferably, the donor template is a DNA donor template, more preferably a double stranded DNA donor template.

Preferably, the sequence of the exogenous polynucleotide of the donor template capable of reconstituting the target gene is a codon-usage optimized sequence, that it is not recognized and/or disrupted by the gene editing agent.

In some embodiments, the donor template does not comprise a reporter gene, e.g., a fluorescent reporter gene or an antibiotic resistance gene.

The donor template according to the invention comprises a knock-in cassette in between two homology arms for HDR integration of the knock-in cassette in the target gene.

In particular, the 5' homology arm (or "left" homology arm) of the donor template has a sequence that is homologous, preferably identical, to a sequence located 5' of the DSB in the target site, preferably contiguous to the DSB, and the 3' homology arm ("right" homology arm) of the donor template has a sequence that is homologous, preferably identical, to a sequence located 3' of the DSB, preferably contiguous to the DSB, in the target site of the cell genome.

When the target site is located within an exon of the target gene, in accordance with preferred embodiments of the invention, the exogenous polynucleotide having sequence capable of reconstituting the target gene has sequence comprising, or consisting of, a sequence homologous to the endogenous sequence of said exon, or to a portion thereof. More preferably, said sequence is a codon-usage optimized sequence, that it is not recognized and/or disrupted by the gene editing agent.

In some embodiments, the exogenous polynucleotide having sequence capable of reconstituting the target gene, or a portion thereof, is part of one of the homology arms.

In some embodiments, the exogenous polynucleotide having sequence capable of reconstituting the target gene, or a portion thereof, is part of the knock-in cassette.

In some embodiments, the target site is within an exon of the target gene and the exogenous polynucleotide having sequence capable of reconstituting the target gene has a first portion whose sequence is a codon-usage optimized sequence corresponding to the endogenous sequence of the portion of exon that is upstream the DSB and a second portion whose sequence is a codon-usage optimized sequence corresponding to the endogenous sequence of the portion of exon that is downstream the DSB, wherein the first portion is part of the left homology arm and the second portion is part of the knock-in cassette.

Such an embodiment is exemplified in Fig. 1 C and D.

In some embodiments, the target site is within an exon of *UBA1* gene and the HAs of the donor template have sequences homologous to sequences of the target gene that are located 5' and 3' of the DSB in said exon. According to preferred embodiments, the target site is within exon 3 of *UBA1* gene and the left and right HAs of the donor template have sequences comprising, or consisting of, SEQ ID NO: 40 and 41, respectively.

In some embodiments, the target site is within an exon of *RPS19* gene and the HAs of the donor template have sequences homologous to sequences of the target gene that are located 5' and 3' of the DSB in said exon. According to preferred embodiments, the target site is within exon 5 of *RPS19* gene and the left and right HAs of the donor template have sequences comprising, or consisting of, SEQ ID NO: 44 and 45, respectively.

In some embodiments, the target site is within an exon of *OGT* gene and the HAs of the donor template have sequences homologous to sequences of the target gene that are located 5' and 3' of the DSB in said exon. According to preferred embodiments, the target site is within exon 12 of *OGT* gene and the left and right HAs of the donor template have sequences comprising, or consisting of, SEQ ID NO: 50 and 51, respectively.

Preferably, the knock-in cassette comprises an exogenous polynucleotide encoding a gene product of interest that is the product of an endogenous gene that is missing and/or altered in the target cells and that it is meant to be added and/or corrected through HDR gene editing of the cells.

Advantageously, the method and kit of the invention can be used as a platform for expressing any gene of interest integrated in a locus that is safe and that takes advantage of the selection approach of the invention, wherein the cells that do not integrate the knock-in cassette or that present undesired outcomes of the gene editing are spontaneously purged out, while cells that correctly integrate the knock-in cassette by HDR are indirectly enriched.

More preferably, the gene product of interest is different from the gene product of the target gene. For example, when the target gene is *UBA1* gene, the gene product of interest is not UBA1.

Preferably, the knock-in cassette comprises an exogenous promoter, operably linked to the exogenous polynucleotide encoding the gene product of interest and driving expression of the gene product of interest.

In preferred embodiments, said promoter is a strong promoter, that induces the expression of high levels of the gene product of interest.

Strong promoters that can be used to drive expression of the gene of interest (GOI), in accordance with preferred, non-limiting, embodiments of the invention are: Spleen Focus-Forming Virus promoter (SFFV), MND promoter, gp91phox promoters¹⁶.

Preferably, said promoter is a Spleen Focus-Forming Virus promoter, SFFV, having sequence comprising or consisting of SEQ ID NO: 37 (or SEQ ID NO: 54 when the gene of interest in the knock-in cassette is in an antisense direction as compared to the transcription of the native gene).

In some embodiments, said promoter is a MND promoter (SEQ ID NO: 55, or its reverse complement when the GOI in the knock-in cassette is in an antisense direction as compared to the transcription of the native gene) or a chimeric gp91phox -SP146 synthetic promoter (SEQ ID NO: 56 or its reverse complement when the GOI in the knock-in cassette is in an antisense direction as compared to the transcription of the native gene).

Advantageously, the method of the invention admits the use of strong and very strong promoters to induce expression of the gene product of interest, in view of the safety profile of the target loci of HDR integration.

In some embodiments the knock-in cassette does not comprise an exogenous promoter as the expression of both the target gene and the gene of interest is driven by the endogenous promoter of the target gene upon integration of the knock-in cassette.

In some embodiments, the knock-in cassette comprises one or more regulatory elements that enable expression of the gene product of interest and/or of the target gene product as separate gene products.

When the expression of the gene of interest is under the control of the same promoter driving expression of the target gene product, the knock-in cassette comprises an IRES or 2A element to separate the expression of the target gene and of the gene of interest.

Preferably, the polynucleotide of the knock-in cassette encoding the gene product of interest is under the control of an exogenous promoter and the knock-in cassette comprises a polyadenylation (polyA) sequence downstream of sequence encoding for the gene product of interest, and optionally a 3' UTR sequence upstream of the polyA.

In some embodiments, the target site is within an exon of the target gene, that is followed by one or more further exons in the endogenous gene. In these and other embodiments, preferably the exogenous polynucleotide having sequence capable of reconstituting the target gene comprises a splicing site and the polynucleotide encoding the gene product of interest is under the control of an exogenous promoter and comprises a polyadenylation signal. In this way, the gene product of interest is transcribed separately from the endogenous gene product of the target gene upon integration of the knock-in cassette. Moreover, the endogenous target gene will be reconstituted with its exons in frame upon integration of the knock-in cassette.

Preferably, the target site is within exon 3 of *UEA1*, and the knock-in cassette comprises, from the 5' end to the 3' end:
an exogenous polynucleotide having sequence capable of reconstituting *UBA1*gene being SEQ ID NO: 36, consisting of a codon-usage optimized sequence corresponding to the endogenous sequence of the portion of exon 3 of *UBA1* that is downstream the DSB in the endogenous gene, with a splicing donor domain (SD);
an exogenous polynucleotide (gene of interest, GOI) encoding for a gene product of interest, under the control of an exogenous promoter, more preferably of a strong viral promoter, most preferably SFFV promoter (SEQ ID NO: 37); and a polyadenylation (polyA) signal, more preferably a bGH polyadenylation (polyA) signal (SEQ ID NO: 39), (see Fig. 4 A "sense").

Optionally, the knock-in cassette for integration in exon 3 of UBA1 by HDR can bear the gene of interest in an antisense direction as compared to the transcription of the native gene, said cassette comprising in sequence from the 5' end to the 3' end: a polyA, preferably having sequences SEQ ID NO: 52, the GOI, and the promoter, preferably having sequences SEQ ID NO: 54 (see Fig. 4 A "antisense").

In the donor template, said knock-in cassettes are between homology arms (HAs) for HDR insertion in the target site of exon 3 of *UBA1* gene and reconstitution of the *UBA1* gene. Preferably, the target site has sequence comprising, or consisting of, SEQ ID NO: 5 and the left and right HA have sequences comprising, or consisting of, SEQ ID NO: 40 and 41, respectively.

In some embodiments, the target site is within last or second-last exon of the target gene. In these and other embodiments, preferably the exogenous polynucleotide having sequence capable of reconstituting the target gene comprises and the polynucleotide encoding the gene product of interest can be under the control of the endogenous promoter upon integration of the knock-in cassette, preferably being separated in the knock in cassette by a IRES or 2A element, more preferably the knock-in cassette including a polyA signal.

Preferably, the target site is within exon 5 of *RPS19* and the knock-in cassette comprises in sequence, from the 5' end to the 3' end:
an exogenous polynucleotide having sequence capable of reconstituting the target gene being SEQ ID NO: 42, consisting of a codon-usage optimized sequence corresponding to the endogenous sequence of the portion of exon 5 of *RPS19* gene that is downstream of the DBS in the endogenous gene and of exon 6 of *RPS19* gene, a P2A domain having sequence SEQ ID NO: 43, an exogenous polynucleotide (gene of interest, GOI) encoding for a gene product of interest, and a polyadenylation (polyA) signal, more preferably a bGH polyA having sequence SEQ ID NO: 39 (see Fig. 4 B).

In the donor template, said knock-in cassette is between homology arms (HAs) for HDR insertion in the target site of exon 5 of *RPS19* gene and reconstitution of the *RPS19* gene. Preferably, the target site has sequence comprising, or consisting of, SEQ ID NO: 1 and the left and right HA have sequences comprising, or consisting of, SEQ ID NO: 44 and 45, respectively.

In further embodiments, the target site is within exon 4 of *RPS19* and the knock-in cassette comprises in sequence, from the 5' end to the 3' end:
an exogenous polynucleotide having sequence capable of reconstituting the target gene being SEQ ID NO: 46 consisting of a codon-usage optimized sequence encoding the portion of exon 4 of *RPS19* gene that is downstream the DSB in the endogenous gene with a splicing donor domain (SD), an exogenous polynucleotide encoding for a gene product of interest, under the control of a strong viral promoter, more preferably SFFV promoter (SEQ ID NO: 37), and a polyadenylation (polyA) signal, more preferably a bGH poly A (SEQ ID NO: 39).

In the donor template, said knock-in cassette is between homology arms (HAs) for HDR insertion in the target site of exon 4 of *RPS19* gene and reconstitution of the *RPS19* gene. Preferably, the target site has sequence comprising, or consisting of, SEQ ID NO: 3 and the left and right HA have sequences comprising, or consisting of, SEQ ID NO: 47 and 48, respectively.

In further embodiments, the target site within exon 12 of *OGT* gene and the knock-in cassette comprises in sequence from the 5' end to the 3' end:
an exogenous polynucleotide having sequence capable of reconstituting the target gene being SEQ ID NO: 49, consisting of a codon-usage optimized sequence encoding the portion of exon 12 of *OGT* gene that is downstream of the DSB in the endogenous gene with a splicing donor domain (SD), and an exogenous polynucleotide encoding for a gene product of interest, under the control of a strong viral promoter, more preferably SFFV promoter (SEQ ID NO: 37), and a polyadenylation (polyA) signal, more preferably a bGH polyA (SEQ ID NO: 39).

In the donor template, said knock-in cassette is between homology arms (HAs) for HDR insertion in the target site of exon 12 of *OGT* gene and reconstitution of the *OGT* gene*.* Preferably, the target site has sequence comprising, or consisting of, SEQ ID NO: 27 and the left and right HA have sequences comprising, or consisting of, SEQ ID NO: 50 and 51, respectively.

The donor template can be delivered to the cell by means of a viral vector.

Preferably the viral vector is an adeno-associated vector (AAV), or an integration-defective lentiviral vector (IDLV).

Said viral vector can be optimized for maximal transduction efficiency and stable constitutive transgene expression in target cells.

Optionally, the donor template can be delivered to the cell by means of a non-viral vector Non-viral vectors are a heterogeneous group of delivery vectors that comprise polyplexes, lipid nanoparticles, non-lipid nanoparticles, virus-like particles or combinations of these. In comparison with viral vectors, this group is characterized by low cytotoxic, immunogenic and mutagenic profiles. Moreover, they also present high cargo capacity.

Most lipids consist of positively charged headgroups which bind with the anionic phosphate groups of nucleic acids via electrostatic interactions to form lipid nanoparticles.

Polyplexes are formed when polyanionic nucleic acids compact with polycationic polymers.

Non-lipid nanoparticles may comprise carbon- or metal- based nanoparticles, examples of which include carbon nanotubes, graphene or carbon quantum dots (CQDs) and gold or iron oxide nanoparticles. Virus-like particles are virus-derived structures made of one or more different molecules with the ability to self-assemble, mimicking the form and size of a viral particle, therefore they maintain the ability to transduce the target cell, but they lack the viral genetic material.

Non-viral delivery systems include DNA transfection such as electroporation, lipid mediated transfection, compacted DNA-mediated transfection; liposomes, immunoliposomes, lipofectin, cationic facial amphiphiles (CFAs) and combinations thereof.

Preferably, the donor template is delivered to cells in a transfected plasmid or as linear or circular naked DNA.

The delivery of the donor template by a vector system according to the present invention may be used alone or in combination with other treatments or components of the treatment.

Preferably, when any of the gene editing agent and/or donor template is introduced into a cell, delivered with a viral vector, according to the method of the invention, the step of transducing the cell with the viral vector includes stimulation of cells in the presence of a human cytokine mix, more preferably for about 22-hour, followed by the addition of the viral particles, preferably for about 14 hours upon transduction.

Preferably, when the cell is transduced a cell with a IDLV viral vector, at least one viral transduction enhancer, more preferably PGE, CsH, CsA or a poloxamer, and/or at least one expansion enhancer, more preferably UM171, UM729, StemRegenin1 (SR1), diethylaminobenzaldehyde (DEAB), LG1506, BIO (GSK3β inhibitor), NR-101, trichostatin A (TSA), garcinol (GAR), valproic acid (VPA), copper chelator, tetraethylenepentamine, and nicotinamide, is added to the cell culture before transduction, according to optimized protocols, for instance as described in WO2013049615, WO2018193118, WO2013127964 and in Delville et al.

According to preferred embodiments of the method of the invention, the cells are further genetically engineered to express an engraftment enhancer. More preferably said engraftment enhancer is CD47 and/or C-X-C chemokine receptor type 4 (CXCR4).

According to preferred embodiments, the cells are cultured in the presence of an inhibitor of senescence, such as inhibitor of MAPK/ERK signaling, an IL-1 inhibitor and/or an NF-κB inhibitor. Preferably, the inhibitor of MAPK/ERK signaling is a MAP3K inhibitor, a MAK2K inhibitor, a MAPK inhibitor, preferably an MKK7 inhibitor, an MKK4 inhibitor, an MKK3/6 inhibitor, an MEK1/2 inhibitor, a JNK inhibitor, a p38 inhibitor, a p53 inhibitor or an ERK inhibitor. Preferably, the inhibitor of p53 activation is a p53 dominant negative peptide, an ataxia telangiectasia mutated (ATM) kinase inhibitor or an ataxia telangiectasia and Rad3-related protein (ATR) inhibitor. In some embodiments, the inhibitor of p53 activation is pifithrin-α or a derivative thereof; KU-55933 or a derivative thereof; GSE56 or a variant thereof; KU-60019, BEZ235, wortmannin, CP-466722, Torin 2, CGK 733, KU-559403, AZD6738 or derivatives thereof; or an siRNA, shRNA, miRNA or antisense DNA/RNA, preferably wherein the inhibitor of p53 activation is GSE56 or a variant thereof.

Preferably, the cells are cultured in the presence of GSE56 and/or of Ad5-E4orf6/7 protein.Preferably the engineered cells obtained from the method of the invention are collected, and optionally resuspended in a freezing medium for further use.

Preferably, after collection, the engineered cells are then washed and resuspended, more preferably at a concentration of 2.5-10 × 10⁶ cells/ml, in a minimum volume of freezing medium (e.g., 20 ml), and cryopreserved under vapor of liquid nitrogen in cryobags or vials.

The cells can be frozen until use.

Preferably the cells of the method or kit of the invention are autologous cell, i.e., cells obtained from a subject, to which the cells are meant to be reinfused, once genetically modified.

The present invention is also directed to pharmaceutical formulations comprising a population of the gene edited cells, obtainable by the method of the invention, or the kit of the invention, and suitable pharmaceutically acceptable excipients.

The term "pharmaceutically acceptable excipient" refers to a non-toxic solid, semisolid, or liquid filler, diluent, encapsulating material, or formulation auxiliary of any conventional type that may optionally be included in the formulations of the invention and that causes no significant adverse toxicological effects to the patient. A pharmaceutically acceptable excipient is essentially non-toxic to recipients at the employed dosages and concentrations and is compatible with other ingredients of the formulation. The number and the nature of the pharmaceutically acceptable excipients depend on the desired administration form.

In a further aspect, the present invention is also directed to the kit or to the gene edited cell obtainable by the method of the invention, or the pharmaceutical formulation thereof, for use as a medicament, preferably for use in the treatment of a genetic disease that can be treated by expressing the gene product of interest in a cell (gene therapy).

The present invention is then also directed to the kit or to the gene edited cell obtainable by the method of the invention, or the pharmaceutical formulation thereof, for use as medicament in gene therapy, preferably in *ex vivo* gene therapy.

The present invention is then directed also to a method of treatment of a disease by gene editing, comprising administering to a subject in need thereof a therapeutic amount of the kit of the invention or of the gene edited cells obtained from the method of the invention, or a pharmaceutical formulation thereof.

A skilled worker would be able to determine appropriate dosage rates. The term "administered" includes delivery by viral or non-viral techniques.

In preferred embodiments, the *ex vivo* engineered cells are administered as part of an autologous cell transplant procedure.

In other preferred embodiments, the engineered cells are administered as part of an allogeneic cell transplant procedure.

Preferably, the subject receiving the cells is subjected to a partial or full myeloablative conditioning regimen, before administration of the cells. Example regimens may involve nonspecific, chemo- or radio-therapeutic treatments.

In some embodiments, the subject has received biological myeloablative or non-myeloablative conditioning regiment (e.g. myeloablative conditioning using an antibody), before administration of the cells.

It should be understood that all the possible combinations of the preferred aspects of the present invention are also described, and therefore similarly preferred.

Examples of preferred embodiments of the present invention and analyses of their efficacy are provided below for illustrative and non-limiting purposes.

### EXAMPLES

### MATERIALS AND METHODS

### Cell culture of human HSPCs

G-CSF or G-CSF + Plerixafor mPB CD34+ HSPCs were purified in house with the CliniMACS CD34 Reagent System (Miltenyi Biotec) from Mobilized Leukopak (AllCells) according to the TIGET-HPCT protocol approved by OSR Ethical Committee and following the manufacturer's instructions. HSPCs were seeded at the concentration of 7.5 ×10^5 cells ml-1 in serum-free StemSpan SFEM supplemented with 100 ill ml-1 penicillin, 100 µg ml-1 streptomycin, 2% glutamine, 300 ng ml-1 hSCF, 300 ng ml-1 hFlt3-L, 100 ng ml-1 hTPO and 10 µM 16,16-Dimethyl Prostaglandin E2 (added at the beginning of the culture), 1 µM SR1 and 35 nM UM171.

Alternatively, for clinical-compliant electroporation, mPB CD34+ HSPCs were cultured in a medium optimized for expansion, which is still under evaluation for patenting and that cannot be disclosed yet.

All cells were cultured in a 5% CO2 humidified atmosphere at 37 °C.

### Vectors

AAV6 donor templates were generated from a construct containing AAV2 inverted terminal repeats, produced at the TIGEM Vector Core (in the case of AAVS1 target locus) or by Genewiz Azenta (for all the other AAV6 vectors) by a triple-transfection method and purified by ultracentrifugation on a cesium chloride gradient.

Design of the AAV6 donor templates carrying homologies for the target loci (all-encompassing an EGFP reporter cassette) are reported in Figure 4.

Vector maps were designed with SnapGene software v.5.0.7 (from GSL Biotech, available at snapgene.com).

### Gene editing of human HSPCs (standard conditions) and analyses

For each condition, from 2.5×10^5 to 7.5×10^5 mPB-derived HSPCs were washed with ten volumes of DPBS without Ca2+ and Mg2+ and electroporated using the P3 Primary Cell 4D-Nucleofector X Kit (Lonza) and program EO-100 after 3 days of culture. HSPCs were electroporated with RNP complex RNPs at a final concentration of 2.5µM (50 pmol gRNA, Synthego). RNP complexes were assembled by incubating at a 1:1.5 molar ratio Streptococcus pyogenes (Sp)Cas9 protein (Aldevron) with pre-annealed synthetic RNA guide (Synthego) for 10min at 25 °C, according to the manufacturer's instructions.

All the sequences of RNA guides used for editing are listed in Table 2.

RNA guide used for editing *AAVS1* gene targets sequence SEQ ID NO: 35.

After editing HSPCs were kept in culture up to seven days after editing. Four days after electroporation HSPCs were collected to extract genomic gDNA for molecular analysis.

### Colony-forming cell (CFC) assays

Colony-forming cell (CFC) assays were performed 24 hrs after editing procedure by plating 400-800 cells in methylcellulose-based medium (MethoCult H4434, StemCell Technologies) supplemented with 100 IU ml-1 penicillin and 100 µg ml-1 streptomycin. Three technical replicates were performed for each condition. Two weeks after plating, colonies were counted.

### Mice

All experiments and procedures involving animals were performed with the approval of the Animal Care and Use Committee of the San Raffaele Hospital and authorized by the Italian Ministry of Health and local authorities accordingly to Italian law. NSG and NBSGW female mice were held in specific pathogen-free conditions.

### CD34+ HSPC xenotransplantation experiments

For xenotransplantation the outgrowths of 7.5 ×10^5 to 1×10^6 HSPCs at the start of the culture (T0) were injected intravenously 24 hrs after editing into sublethally irradiated NSG mice (180-200 cGy) or in NBSGW mice. Matched numbers of HSPCs were seeded at day 0 of culture for each experimental group in order to transplant in each mouse the same number of culture-initiating HSPCs. Mice were randomly distributed to each experimental group. Human CD45+ cell engraftment and the presence of edited cells were monitored by serial collection of blood (approximately every 2 to 3 weeks) from the retroorbital plexus and, at the end of the experiment (12-16 weeks after transplantation), BM and SPL were collected for end-point analyses, including florescence-activated cell sorting (FACS) of hematopoietic lineages in some experiments.

### Flow cytometry

Immunophenotypic analyses were performed by flow cytometry using Canto II (BD Pharmingen). Cells were stained for 15' at 4 °C with antibodies in a final volume of 100 µl and then washed with DPBS + 2% heat-inactivated FBS. Single stained and fluorescence-minus-one-stained cells were used as controls. The Live/Dead Fixable Dead Cell Stain Kit (Thermo Fisher) or 7-aminoactinomycin D (Sigma Aldrich) were included during sample preparation according to the manufacturer's instructions to identify dead cells.

### Molecular analyses

For molecular analyses, gDNA was isolated with QIAamp DNA Micro Kit (QIAGEN) according to the manufacturer's instructions.

Nuclease activity was measured using a mismatch-sensitive endonuclease T7 assay (New England Biolabs) on PCR-based amplification products of the targeted locus, as previously described. Digested DNA fragments were resolved and quantified by capillary electrophoresis on 4200 TapeStation System (Agilent) according to the manufacturer's instructions.

For HDR digital droplet PCR (ddPCR) analysis, 5-50ng of gDNA were analyzed using the QX200 Droplet Digital PCR System (Bio-Rad) according to the manufacturer's instructions. HDR ddPCR primers and probes were designed to fully cover the junction between the vector sequence and the targeted locus with a single amplicon. Human TTC5 (Bio-Rad) was used for normalization. The percentage of cells harboring biallelic integration was calculated with the following formula: (no. of target gene+ droplets/no. of of TTC5+ droplets×200)-percentage of GFP+ cells. The percentage of monoallelic integration was then calculated with the following formula: percentage of GFP+ cells-percentage of cells with biallelic integration.

### EXAMPLE 1

Each gene of Table 1 has been tested in vitro as candidate target gene in accordance with the method of the invention.

According to the experimental design (Fig. 2A) mobilized-peripheral blood HSPCs CD34+ (m-PB CD34+ HSPCs) were thawed and pre-cultured for 3 days in standard conditions (cytokine-added stem span medium, according to Schiroli et al⁴).

On the third day after thawing (day 0), the RNP complex (candidate gRNA plus Cas9 protein) was delivered to the cell through electroporation (Lonza Nucleofector). Three guide RNAs were tested for each condition, except for *UBLA4* and *RPS19* for which two and four different guide RNAs were tested, respectively. The day after editing (day 1), 600 cells per condition were plated into classic methocult medium for clonogenic in vitro assay (CFA) in 3 technical replicates for each condition. 4 days after editing (day 4, at which the higher value of edited cells is presumably registered before occurrence of any counterselection event), the percentage of indels in the bulk culture was evaluated through an enzymatic non-homologous end joining assay (T7 endonuclease assay). Results are shown in Fig. 2B. 14 days after editing the absolute number of red and white colonies obtained from the CFA was counted. Results are shown in Fig. 2C.

For each experiment beta-2 microglobulin human (Beta2M) knock-out was used as control, since Beta2M knock out is known not to preclude the cell culture composition and clonogenic output of HSPCs *in vitro* (Beta2M gRNA target sequence is SEQ ID NO: 34).

*RPS19* gene is an autosomal genes, which is implied in the pathogenesis of Blackfan-Diamond anemia, when mutated. A proof of concept of the haploinsufficiency of this gene has been reported in literature¹⁴. 4 different guide RNAs were designed targeting exons 4, 5 and 6 of this gene: the ones targeting exon 4 and the one targeting exon 5 (respectively RPS19_3 and RPS19_1 in Table 2) have displayed a good cutting efficiency (45%) and were able to impair the clonogenic output of healthy donors' HSPCs in vitro in three biological replicates (see **Figs. 2B and 2C**).

Essential X-linked genes are, by definition, haploinsufficient in males and, in most cases, also in females (if they don't escape lyonization). 10 different genes on chromosome X for which there is evidence of haploinsufficiency in HSPCs or for which haploinsufficiency is expected (i.e. *ABCB7*, *AIFM1*, *DKC1*, *FLNA*, *HCCS*, *MED12*, *OGT*, *PGK1*, *UBLA4*, *UBA1*) have been tested, each with three different gRNAs (except for UBLA4, tested with two gRNAs); for each of them the cutting efficiency (**Fig. 2B**) and the impairment of the clonogenic potential of HSPCs in culture (**Fig. 2C**) were evaluated on three different biological replicates, 2 performed on male and 1 on female donors' HSPCs.

Among the X-linked genes, a promising candidate resulted to be Ubiquitin like modifier activating enzyme 1 (*UBA1*), a gene which is involved in proteasomal degradation patterns. Point mutations inducing an impairment in the cytoplasmic isoform of this gene are associated to an acquired inflammatory syndrome called VEXAS (Vacuoles, E1 enzyme, X-linked, Autoinflammatory, Somatic). When completely knocking out the function of both nuclear and cytoplasmic UBA1 isoforms with an efficient guide RNA, such as gRNA targeting the exon 3 (gRNA targeting sequence SEQ ID NO: 5 in Table 2), having 70-80% efficiency, the clonogenic output was completely impaired, both in 1 female and in 2 male HSPCs donors (**Fig. 2C**). Other candidates genes on the X chromosome displaying high efficiency are *AIFM1*, *DKC1* and *OGT* genes, for which one gRNA per each gene was identified ((gRNAs targeting sequences 12, 16 and 27, respectively in Table 2), obtaining median % of indels in culture of 70%, 60% and 58%, respectively, and impairing significantly the clonogenic output (yellow arrows in **Fig.2C**).

### EXAMPLE 2

Five promising candidate genes (UBA1, RPS19, AIFM1, DKC1 and OGT genes) and the respective best-performing gRNAs, as assessed in Example 1, were selected to verify that the clonogenic impairment *in vitro* correlates with the impairment of long-term engraftment *in vivo.* To evaluate this, a set of identical experiments was performed (**Fig. 3A**), in which each guide gRNA was used for *in vitro* editing in HSPCs, as described in Example 1, then, the day after editing, the outgrowths of same number of HSPCs at the start of the culture (i.e. T0, 750,000 per mouse ⁵) were transplanted into 5 NSG mice per each group, 6 hours after sublethal irradiation of the mice (180 Gy). Each experiment had a mock-edited control (edited in a neutral locus, the genomic safe harbor *AAVS1*).

Blood samples were collected from mice every two weeks starting from week 5 after transplantation. At sacrifice (week 13) blood, spleen and bone marrow were collected from each mouse. All the samples at all timepoints were evaluated for human engraftment and cell composition by FACS and for indel's percentage on genomic DNA extracted from pellet.

As for the engraftment of human cells, evaluated as the percentage of cells staining positively for the surface antigen hCD45 at FACS, as expected, there was an initial tendency to a lower engraftment at earlier timepoints (weeks 5 and 7 after editing) in the experimental groups as compared to control group, but this difference was lost at later timepoints (after week 9), when there was a plateau of human engraftment for all groups. Beyond week 9, the human engraftment (as typical for xenotransplantation into this mouse model) progressively lowered over time and at sacrifice (week 13) there were no more differences in human engraftment neither in peripheral blood, spleen and bone marrow (**Fig. 3B**).

As for the percentage of indels in the human graft, the results show a clear tendency towards a stable/slight increase in the fold of indels for the AAVS1 group and a tendency to progressive reduction in the case of the candidate genes.

As for *UBA1* gene (1 gRNA tested) and *RPS19* gene (2 gRNAs tested), in both cases a significant reduction was observed in the % of indels over time from the input (*in vitro* % indels, at day 4 after editing) that were of 65%, 42% and 25% for UBA1 gRNA and for RPS 19 gRNAs 1 and 3, respectively, and the final % of indels in the spleen and bone marrow at the moment of sacrifice of the mice (13 weeks after transplantation), that were for all candidate genes less than 5%, differently from what happened in the control group for which the % of indels remained more or less stable over time (20-35%).

As for the other three candidate genes, *AIFM1*, *DKC1* and *OGT* genes (one guide RNA tested for each gene), an analogous experiment showed a similar trend in terms of human engraftment over time (slight difference between experimental and control group at week 5 on peripheral blood, lost at later time points in blood, spleen and bone marrow, **Fig. 3B**). As for indels' percentage, the tendency towards a counterselection as compared to the input in vitro was much more evident for *OGT* gene as compared to the other two tested genes.

Indel's fold growth, obtained when normalizing for in vitro editing efficiency, show a clear tendency towards a stable/slight increase in the fold over the in vitro sample for the *AAVS1* group and a tendency to progressive reduction in the case of *UBA1*, *RPS19* and *OGT* genes (**Fig. 3C**).

This is indirect evidence of the disadvantage of HSPCs bearing indels in the selected genes in terms of long-term engraftment. (This evidence is not being reflected in terms of percentage of engrafted human cells over time, because the non-edited cells are expected to quickly repopulate the bone marrow niche of the mice, evening out the differences between groups).

The trend observed over time confirms counterselection in the target genes as compared to the control. These loci have therefore proven to be suitable for the negative selection approach of the invention, providing for spontaneous long-term purge out of HSPCs bearing indels *in vivo.*

### EXAMPLE 3

Knock-in cassettes have been prepared to reconstitute the haploinsufficient target gene that is knocked-out *in vivo* by gene editing agents, restoring the clonogenic capacity *in vitro* and the long-term engraftment potential *in vivo* of edited HSPCs, expressing at the same time a gene of interest (GOI).

A knock-in cassette was prepared for reconstituting *UBA1* gene (**Fig. 4A**) after disruption by gene editing with a gRNA targeting exon 3 of *UBA1* gene (gRNA target sequence SEQ ID NO: 5); inside the homology arms (left arm SEQ ID NO: 40 and right arm SEQ ID NO: 41), the knock-in cassette (sense cassette in **Fig. 4A**) comprises in sequence from the 5' end to the 3' end: the final part of exon 3 of *UBA1* gene that is 3' of the break in the endogenous gene (codon-usage optimized, in order not to be re-cut by the guide RNA after HDR) with a splicing donor domain (SD) (SEQ ID NO: 36), and a cassette for expressing a gene of interest (GOI cassette), consisting of a strong viral promoter (Spleen Focus-Forming Virus promoter, SFFV, SEQ ID NO: 37), EGFP (SEQ ID NO: 38) mocking the gene of interest, and a bGH (Bovine Growth Hormone) polyadenylation (polyA) signal (SEQ ID NO: 39). A different form of the knock-in cassette was also designed, bearing the GOI cassette in an antisense direction as compared to the transcription of the native gene (anti-sense cassette in **Fig. 4A**, with polyA, GOI and promoter, respectively of SEQ ID NO: 52, 53 and 54, in antisense direction).

A knock-in cassette was prepared for reconstituting *RPS19* gene (**Fig. 4B**) after disruption by gene editing with a gRNA cutting in the terminal region of *RPS19* gene (gRNA targeting sequence SEQ ID NO: 1, in the final part of exon 5) e; inside the homology arms (left arm SEQ ID NO: 44 and right arm SEQ ID NO: 45), the knock-in cassette comprises in sequence from the 5' end to the 3' end: the final part of the *RPS19* gene (end of exon 5 and coding region of exon 6, codon-usage optimized, in order not to be re-cut by the guide RNA after HDR, SEQ ID NO: 42) and the gene of interest (mocked by EGFP, SEQ ID NO: 38 , followed by a bGH polyA domain, SEQ ID NO: 39) under the control of the endogenous promoter of the *RPS19* gene, after reconstitution of the same via HDR, and separated from the coding region of the *RPS19* gene by a P2A domain (SEQ ID NO: 43).

A further knock-in cassette was also prepared for reconstituting RPS19 gene upon targeting of exon 4 with the relative gRNA (SEQ ID NO: 3), said knock-in cassette being designed similarly to that of *UBA1* locus, keeping the EGFP under the control of the SFFV promoter and recoding only the final part of exon 4 of RPS19 gene inside the knock-in cassette (codon-usage optimized RPS19 exon 4, SEQ ID NO: 46), inside the homology arms (left arm SEQ ID NO: 47 and right arm SEQ ID NO: 48).

A knock-in cassette was also prepared for reconstituting *OGT* gene, with a design similar to that of the knock-in cassettes for *UBA1* and *RPS19*-exon 4.

### EXAMPLE 4

HDR editing experiments were performed using the guide RNA knocking out exon 3 of *UBA1* gene and the one knocking out exon 5 of *RPS19* gene.

For each of these two loci, AAV6 constructs were designed bearing homology arms for the regions disrupted by the respective guide RNAs.

To evaluate the efficacy of these vectors in restoring the clonogenic capacity, in vitro experiments were performed with a design similar to what described above (**Fig.5** **A**), but in this case for each guide RNA the test included: a KO only condition (called RNP), a condition in which vector was simply added without RNP delivery (called AAV6 condition) and a full HDR condition (called HDR) with the respective AAV6 used as donor template in a classical HDR platform (i.e. added 15 minutes after RNP electroporation) at the standard MOI of 20000. As a control, in the AAVS1 group, a standard PGK-GFP AAV6 was used at the same dose. In the HDR conditions, editing efficiency was assessed through FACS analysis (GFP expression within the CD90+ compartment 7 days after editing) and the clonogenic restoring capacity was evaluated both in terms of colonies' count and in terms of GFP expression of cells pooled from colonies. For each experiment, two biological replicates were performed, one of the two with adding the so-called genetic suppressor element (GSE), an editing enhancer that works as temporary inhibitor of TP53 activation ⁴ to the electroporation mix.

For *UBA1* gene, the results in terms of HDR efficiency (measured as % of GFP positive cells in vitro) with both sense and anti-sense constructs were similar or slightly better than what normally ob served for HDR platform in standard condition, with HDR reaching 20% of AAVS1 edited Cd90+ cells (the most primitive among HSPCs), and 30% and 35% with the sense and anti-sense UBA1 constructs, respectively.

As for the number of colonies, while *AAVS1* RNP and AAV6 conditions did not impair significantly the colonies' count as compared to the untreated condition (being the only impairment in this case due to the direct toxicity of electroporation and AAV6 itself), in the case of the *UBA1* conditions, the strong impairment seen in the RNP condition (<25 total colonies as compared to nearly 100 in the untreated condition), was partially rescued in the HDR conditions (45 and 40 total colonies, with sense and anti-sense constructs, respectively).

As for GFP expression in colonies, while in the *AAVS1* group the % of GFP positive cells remained the same to what seen in culture at day 7, as for *UBA1* gene for both constructs there was an increase up to nearly 80% of GFP positive cells in colonies, implying that the HSPCs bearing the intended edit have been responsible for the formation of most of the colonies that were able to be formed (**Fig. 5 B)**.

As for *RPS19* gene, the GFP positive Cd90+ cells in culture at day 7 were around 30% and 65% in the *AAVS1* and *RPS19* group, respectively.

In terms of clonogenic output, in the *RPS19* group there was a partial rescue in the number of colonies when comparing the RNP group (<25) with the HDR group (40).

As for GFP expression, in *AAVS1*-edited colonies the percentage of GFP positive cells was stable as compared to the in vitro culture, while it was furtherly increased to up to 80% as compared to the Cd90+ population in culture in the *RPS19* group (**Fig. 5** **C**).

These results provide proof of concept of selective advantage of HDR edited cells as compared to NHEJ-edited cells in vitro.

### EXAMPLE 5

With the most promising candidate (*UBA1*), it was decided to move on with the consequent step of evaluating if the selective advantage shown in vitro for HDR edited cells was maintained in vivo.

1 million HSPCs at T0 per mouse were edited and transplanted the day after into NOD.Cg-KitW-41J Tyr + Prkdcscid Il2rgtm1Wjl/ThomJ (NBSGW) mice. Groups of treatment included: an RNP only group and a full HDR group for each locus/donor template tested (*AAVS1*, *UBA1* sense and antisense constructs). 30 NBSGW female mice (6 per group) were used, without need for irradiation prior to transplantation.

Blood samples were collected from mice every 2 to 3 weeks starting from week 5 after transplantation and at sacrifice (week 13) also spleen and bone marrow have been collected. (**Fig. 6A**).

Part of the cells were kept in vitro for ddPCR evaluation of HDR efficiency with a specific probe assay, that showed 4 days after editing 1.1 of average edited copy per cell in the AAVS1 HDR HSPCs and 0.4 and 0.6, respectively, in the sense and anti-sense UBA1 HDR conditions (**Fig. 6C**).

As for human engraftment, at sacrifice (always measured as % of hCD45 positive cells at FACS), in all organs examined (blood, spleen and bone marrow), the *UBA1* RNP condition was the one with the lowest engraftment overall (<0.1%), confirming the engraftment impairment of *UBAI*-KO cells (**Fig. 6B**). Moreover, engraftment in UBA1 HDR conditions was higher as compared to that of the control group (AAVS1 HDR condition). As for the GFP expression by FACS in human cells, despite the apparent lower editing efficiency shown *in vitro* by ddPCR assay (left graph, Fig. 6C), the *UBA1*-edited GFP+ cells were significantly enriched *in vivo* as compared to the *AAVS1* group (>80% vs 30-50%) in blood, spleen and bone marrow (**Fig. 6C**, right graphs).

This experiment provides the proof of concept for effective selection of HDR-edited cells in haploinsufficient loci, such as *UBA1*, upon transplantation.

### BIBLIOGRAPHY

1. Ferrari, G., Thrasher, A. J. & Aiuti, A. Gene therapy using haematopoietic stem and progenitor cells. Nat Rev Genet 22, 216-234 (2021).
2. Wu, Y. et al. Highly efficient therapeutic gene editing of human hematopoietic stem cells. Nat Med 25, 776-783 (2019).
3. Genovese, P. et al. Targeted genome editing in human repopulating haematopoietic stem cells. Nature 510, 235-240 (2014).
4. Schiroli, G. et al. Precise Gene Editing Preserves Hematopoietic Stem Cell Function following Transient p53-Mediated DNA Damage Response. Cell Stem Cell 24, 551-565.e8 (2019).
5. Ferrari, S. et al. Efficient gene editing of human long-term hematopoietic stem cells validated by clonal tracking. Nat Biotechnol 38, 1298-1308 (2020).
6. Wang, J. et al. Homology-driven genome editing in hematopoietic stem and progenitor cells using ZFN mRNA and AAV6 donors. Nature Biotechnology (2015) doi: 10.1038/nbt.3408.
7. Dever, D. P. et al. CRISPR/Cas9 β-globin gene targeting in human haematopoietic stem cells. Nature (2016) doi:10.1038/nature20134.
8. De Ravin, S. S. et al. CRISPR-Cas9 gene repair of hematopoietic stem cells from patients with X-linked chronic granulomatous disease. Science Translational Medicine (2017) doi:10.1126/scitranslmed.aah3480.
9. Schiroli, G. et al. Preclinical modeling highlights the therapeutic potential of hematopoietic stem cell gene editing for correction of SCID-X1. Science Translational Medicine (2017) doi:10.1126/scitranslmed.aan0820.
10. Ferrari, S. et al. Choice of template delivery mitigates the genotoxic risk and adverse impact of editing in human hematopoietic stem cells. Cell Stem Cell 29, 1428-1444.e9 (2022).
11. Allen, F. et al. Predicting the mutations generated by repair of Cas9-induced double-strand breaks. Nat Biotechnol 37, 64-72 (2019).
12. Hanlon, K. S. et al. High levels of AAV vector integration into CRISPR-induced DNA breaks. Nat Commun 10, 4439 (2019).
13. Allen, A. G. et al. A highly efficient transgene knock-in technology in clinically relevant cell types. Nat Biotechnol 42, 458-469 (2024).
14. Bhoopalan, S. V. et al. An RPS19-edited model for Diamond-Blackfan anemia reveals TP53-dependent impairment of hematopoietic stem cell activity. JCI Insight 8, (2023).
15. De Ravin, S.S., Liu, S., Sweeney, C.L. et al. Lentivector cryptic splicing mediates increase in CD34+ clones expressing truncated HMGA2 in human X-linked severe combined immunodeficiency. Nat Commun 13, 3710 (2022).
16. Chiriaco M, Farinelli G, et al. Dual-regulated lentiviral vector for gene therapy of X-linked chronic granulomatosis. Mol Ther. 22(8):1472-1483 (2014)

### SEQUENCES

Sequences disclosed in conjunction with the present invention are enclosed and also displayed in Table 2 (SEQ ID NO: 1-34, gRNAs target sequences) and hereafter.
SEQ ID NO: 34 (Beta2M gRNA target sequence)
   agatagttaagtggggtaag
SEQ ID NO: 35 (AAVS1 gRNA target sequence)
   gtcaccaatcctgtccctag
SEQ ID NO: 36 (codon-usage optimized UBA1 exon 3 portion with splicing donor domain) tcccgacaattgtaag
SEQ ID NO: 37 (SFFV promoter) 
gtaacgccattttgcaaggcatggaaaaataccaaaccaagaatagagaagttcagatcaagggcgggtac
atgaaaatagctaacgttgggccaaacaggatatctgcggtgagcagtttcggccccggcccggggccaag
aacagatggtcaccgcagtttcggccccggcccgaggccaagaacagatggtccccagatatggcccaacc
ctcagcagtttcttaagacccatcagatgtttccaggctcccccaaggacctgaaatgaccctgcgcctta
tttgaattaaccaatcagcctgcttctcgcttctgttcgcgcgcttctgcttcccgagctctataaaagag
ctcacaacccctcactcggcgcgccagtcctccgacagactgagtcgcccggg
SEQ ID NO: 38 (EGFP) 
atggtgagcaagggcgaggagctgttcaccggggtggtgcccatcctggtcgagctggacggcgacgtaaa
cggccacaagttcagcgtgtccggcgagggcgagggcgatgccacctacggcaagctgaccctgaagttca
tctgcaccaccggcaagctgcccgtgccctggcccaccctcgtgaccaccctgacctacggcgtgcagtgc
ttcagccgctaccccgaccacatgaagcagcacgacttcttcaagtccgccatgcccgaaggctacgtcca
ggagcgcaccatcttcttcaaggacgacggcaactacaagacccgcgccgaggtgaagttcgagggcgaca
ccctggtgaaccgcatcgagctgaagggcatcgacttcaaggaggacggcaacatcctggggcacaagctg
gagtacaactacaacagccacaacgtctatatcatggccgacaagcagaagaacggcatcaaggtgaactt
caagatccgccacaacatcgaggacggcagcgtgcagctcgccgaccactaccagcagaacacccccatcg
gcgacggccccgtgctgctgcccgacaaccactacctgagcacccagtccgccctgagcaaagaccccaac
gagaagcgcgatcacatggtcctgctggagttcgtgaccgccgccgggatcactctcggcatggacgagct
gtacaagtaa
SEQ ID NO: 39 (bGH polyA) 
ctgtgccttctagttgccagccatctgttgtttgcccctcccccgtgccttccttgaccctggaaggt
gccactcccactgtcctttcctaataaaatgaggaaattgcatcgcattgtctgagtaggtgtcattc
tattctggggggtggggtggggcaggacagcaagggggaggattgggaagacaatagcaggcatgctg
gggatgcggtgggctctatgg
SEQ ID NO: 40 (left HA UBA1 exon 3 donor template) 
gtgcaaatcagaccacctgggttcagatcttaggtctgacagctgtgttaccctgggcaagtcctcac
ctttctgtgcctgtcccctctttgctgtaaaatgggggtactaatacttaagtcttcccttcccccac
agttgctactaacaaacagaaaaacggtacccatgtgctccagggtcacctctgacctttttttcctc
cagatgtccagctcgccgctgtccaagaaacgtcgcgtgtccgggcctgatccaaagccgggttctaa
ctgctcccctgcccagtccgtgttgtccgaagtgccctcggtgccaaccaacgtgagtgtcctctccg
tggagactggcagacgaggtggtgggtgggaaagtcttttgtatcactgtctgtctatccatgctcca
ctcctgtgtgtctccctaaacttgttcttttcctctattcctagggaatggccaagaacggcagtgaa
gcagacatagacgagggcctttac
SEQ ID NO: 41 (right HA UBA1 exon 3 donor template) 
tggggccaaggccgggctgaggggtgtggaatgggacattgagaggataaggttgggtggggcaggcc
ctgacctagagtaccccctaacctggcaggtatgtgttgggccatgaggcaatgaagcggctccagac
atccagtgtcctggtatcaggcctgcggggcctgggcgtggagatcgctaagaacatcatccttggtg
gggtcaaggctgttaccctacatgaccagggcactgcccagtgggctgatctttcctcccaggtacct
cttcctagcacccttccccctttcccccttcccgaggcaccactgttcccggtgccacagccatttca
tctttttccctactgcacacccttacagttctacctgcgggaggaggacatcggtaaaaaccgggccg
aggtatcacagccccgcctcgctgagctcaacagctatgtgcctgtcactgcctacactggacccctc
gttgaggacttccttagtggtttccaggtatcttgggggtactacccagccttctgcccagttttctc
agagcccatctctggtttattcagtagtgttcaatattgatttaatgggtcggcctgaatgtcaggtt
ttgtgctgggggactagagtatgcaggcagcagttagcccctggccctgccttttcttttcttctttt
tttttttttgagacggagtcttgctctgtcgcccaagctagagtgcagtggcgcgatctcggctcact
gcaagctacacctcctgggttcacgccattctcctgcctcagcctcccgagt
SEQ ID NO: 42 (codon-usage optimized RPS19 exon 5 end and exon 6) aggtggccgccgctaacaagaagcac
SEQ ID NO: 43 (furin site and P2A domain)
   aggtggccgccgctaacaagaagcac
SEQ ID NO: 44 (left HA RPS19 exon 5 donor template) 
cttgaggcccggtcatcaattccccaacgaatggtcctgcatagtctgcccagcccctcaggcccctc
ctatcagaggcaggcaggaggggattctgcagaaaagcaaacagcacggggcctcaccccgacctctc
tggctgactagcccaggctccaggagggtaatttagcgactatctgctttcattagcctgctcgttaa
cttttcccaattgatttttcggggcttttgatctaaatgcttgcacaaacaacaccccgtcagctccc
aggggggctcccactactgcccccagctcgttagaatgcacctgactagggccctcagtgggacttgg
ctggcggcaggtggctttttgagaagcctggctcacagccaggagggaaggggctgagaacaggacct
gtgctcactggggcctgcatgacccttccctccccacagcggccgcaaactgacacctcagggacaaa
gagatctggacagaatcgccggac
SEQ ID NO: 45 (right HA RPS19 exon 5 donor template) 
aggtaaggcctgcgtttggggtggggctgggtcccttagtcgctgcccaagcatttccaaagcccata
ctttgtcaggtagacttatttccttctctggagggcacagcccagggtgctggtggggtcagaggagg
gctgcccagagacaggagaaaggacagtcctgtctgacttttcctgttggtgctgcttagaaatttgg
aggcaggctggctgtggtgtctcacccctgtaatcccagcactttgggatgccgaggcaggcgaatta
tctgaggtcaggagttcaagaccattctgaccaacatggagaaaccctgtctctactaaaaatacaaa
attagccgggtgtggtggcacattcctgtaatctcagctactggggaggctgaggcacaaaaatcact
tgaacccgggaggtggaggctgcggtgagccaagatcatgccattgcaccccagcctgggcaacaaga
gtgaaactccatctcaaaaaaaaa
SEQ ID NO: 46 (codon-usage optimized RPS19 exon 4)
   aggtggccgccgctaacaagaagcac
SEQ ID NO: 47 (left HA RPS19 exon 4 donor template) 
cttgaggcccggtcatcaattccccaacgaatggtcctgcatagtctgcccagcccctcaggcccctc
ctatcagaggcaggcaggaggggattctgcagaaaagcaaacagcacggggcctcaccccgacctctc
tggctgactagcccaggctccaggagggtaatttagcgactatctgctttcattagcctgctcgttaa
cttttcccaattgatttttcggggcttttgatctaaatgcttgcacaaacaacaccccgtcagctccc
aggggggctcccactactgcccccagctcgttagaatgcacctgactagggccctcagtgggacttgg
ctggcggcaggtggctttttgagaagcctggctcacagccaggagggaaggggctgagaacaggacct
gtgctcactggggcctgcatgacccttccctccccacagcggccgcaaactgacacctcagggacaaa
gagatctggacagaatcgccggac
SEQ ID NO: 48 (right RPS19 exon 4 donor template) 
aggtaaggcctgcgtttggggtggggctgggtcccttagtcgctgcccaagcatttccaaagcccata
ctttgtcaggtagacttatttccttctctggagggcacagcccagggtgctggtggggtcagaggagg
gctgcccagagacaggagaaaggacagtcctgtctgacttttcctgttggtgctgcttagaaatttgg
aggcaggctggctgtggtgtctcacccctgtaatcccagcactttgggatgccgaggcaggcgaatta
tctgaggtcaggagttcaagaccattctgaccaacatggagaaaccctgtctctactaaaaatacaaa
attagccgggtgtggtggcacattcctgtaatctcagctactggggaggctgaggcacaaaaatcact
tgaacccgggaggtggaggctgcggtgagccaagatcatgccattgcaccccagcctgggcaacaaga
gtgaaactccatctcaaaaaaaaa
SEQ ID NO: 49 (codon-usage optimized OGT exon 12 plus SD) 
cactcaatgttgtacccattgagccacggatttcgcaaagcaatagcagaacgtcatgggaatttatg
tctagacaaggtgtgatt
SEQ ID NO: 50 (left HA OGT exon 12) 
cttccattcataaggtactactgtttattataatatgtgcagtttaacacctaaaatttaacttttgg
aaattttttaccatcctgctttatttattttccaggattcagggaatattccagaagccatagcttct
taccgcacggctctgaaacttaagcctgattttcctgatgcttattgtaacttggctcattgcctgca
ggtaaagaataacaggccagtaattggctctcagtgttgtaatagctttttaattgttgtatgccata
agaatccaagcctgactggaaatagtatttttaataggctatctgacattactttaggccaaagacat
atcaaatattaaatcctgggataggactttctggataataacttgtttttgctttctctagattgtct
gtgattggacagactatgatgagcgaatgaagaagttggtcagtattgtggctgaccagttagagaag
aataggttgccttctgtgcatcct
SEQ ID NO: 51 (right OGT exon 12) 
cttttgttttaatctttttgttgtaaactaaaacacaaatacagaaaactgtaaaaatcaaatctgtg
gcttaatgaacgattataaagtgaacaccacccaagtcgagaaataaaacttttgtgggccacttcag
aaccccttccattgtgtcctgagtataaccccctgcacttcataagacggaatccttaactccttcac
ttaaacactaatgatagactgatggtacttttctttaagttgaaagccataatttttttttcttttat
cttttttttcttttttttttgagacggggtcttgctctgtcgcccaggctggagtgcagcggtgtgat
catggcttactgcagccttgacctccccgagcttaggtgatcctcccacctcagcctcctgagtagct
gggactacaggcgcatgccaccacacctaattttgtgttttttgtagagatggggttttgctatgttg
cccaagctggtcttgaacctctgggctcaagtgatctgcccacctcgcccggccaaagtgctgggatt
acgggtgtgagccaccgcgcctggccagccatcatgtttaatggagtgcttggtattttgtattcacc
tttgagaattagagcagtacttgatcaaggattggatcaaaggttggataatagattggagtgctagg
tgtactttttttttttttttgagacatagtgtctctgtcatccagactgcagtgcagtggtgccatct
cagctcattgcaacctctgtctcccaggttaaagtgatgctcgtgccttagcttcccgaggagctagg
attacaggcgtgtgccaccacgcctggctaatttttgtatttttagtagagatagggctttgccatgc
taggctggtctcgaaa
SEQ ID NO: 52 (bGH polyA antisense) 
ccatagagcccaccgcatccccagcatgcctgctattgtcttcccaatcctcccccttgctgtcctgc
cccaccccaccccccagaatagaatgacacctactcagacaatgcgatgcaatttcctcattttatta
ggaaagggcagtgggagtggcaccttccagggtcaaggaaggcacgggggaggggcaaacaacagatg
gctggcaactagaaggcacag
SEQ ID NO: 53 (EGFP antisense) 
ttacttgtacagctcgtccatgccgagagtgatcccggcggcggtcacgaactccagcaggaccatgt
gatcgcgcttctcgttggggtctttgctcagggcggactgggtgctcaggtagtggttgtcgggcagc
agcacggggccgtcgccgatgggggtgttctgctggtagtggtcggcgagctgcacgctgccgtcctc
gatgttgtggcggatcttgaagttcaccttgatgccgttcttctgcttgtcggccatgatatagacgt
tgtggctgttgtagttgtactccagcttgtgccccaggatgttgccgtcctccttgaagtcgatgccc
ttcagctcgatgcggttcaccagggtgtcgccctcgaacttcacctcggcgcgggtcttgtagttgcc
gtcgtccttgaagaagatggtgcgctcctggacgtagccttcgggcatggcggacttgaagaagtcgt
gctgcttcatgtggtcggggtagcggctgaagcactgcacgccgtaggtcagggtggtcacgagggtg
ggccagggcacgggcagcttgccggtggtgcagatgaacttcagggtcagcttgccgtaggtggcatc
gccctcgccctcgccggacacgctgaacttgtggccgtttacgtcgccgtccagctcgaccaggatgg
gcaccaccccggtgaacagctcctcgcccttgctcaccat
SEQ ID NO: 54 (SFFV promoter antisense) 
cccgggcgactcagtctgtcggaggactggcgcgccgagtgaggggttgtgagctcttttatagagct
cgggaagcagaagcgcgcgaacagaagcgagaagcaggctgattggttaattcaaataaggcgcaggg
tcatttcaggtccttgggggagcctggaaacatctgatgggtcttaagaaactgctgagggttgggcc
atatctggggaccatctgttcttggcctcgggccggggccgaaactgcggtgaccatctgttcttggc
cccgggccggggccgaaactgctcaccgcagatatcctgtttggcccaacgttagctattttcatgta
cccgcccttgatctgaacttctctattcttggtttggtatttttccatgccttgcaaaatggcgttac
SEQ ID NO: 55 (MND promoter) 
ctgcatttatttagtctccagaaaaaggggggaatgaaagaccccacctgtaggtttggcaagctagg
atcaaggttaggaacagagagacagcagaatatgggccaaacaggatatctgtggtaagcagttcctg
ccccggctcagggccaagaacagttggaacagcagaatatgggccaaacaggatatctgtggtaagca
gttcctgccccggctcagggccaagaacagatggtccccagatgcggtcccgccctcagcagtttcta
gagaaccatcagatgtttccagggtgccccaaggacctgaaatgaccctgtgccttatttgaactaac
caatcagttcgcttctcgcttctgttcgcgcgcttctgctccccgagctcaataaaagagcccaggg
SEQ ID NO: 56 (chimeric gp91phox- SP146 synthetic promoter)

cagaaaaggagaagtaggagccaagatttccaaactctgtggttgccttgccaagatttccaaactct
gtggttgccttgcagaaaaggagaagtaggagaagcgacttcctctttccagaagcgacttcctcttt
ccagaggaagagggcggaggctcacaaggcaaccacagagtttggaaatcttggaagcgacttcctct
ttccagcagaaaaggagaagtaggagaagcgacttcctctttccaggtccgccctcgctagatcttta
ttctgtttttaaattttttatttttatattttacctttaaaacatttttgttaaaagctaagacacaa
acatacacattagcctaggcccacacaaagtcaggatcattaatatcactgtcttccacctccacatt
ttgccccactggacagtcttcaagggcactaacatgcatgaagctgtcatctatgacagcaatgcatt
cttctggaataccttctgaaggacctgcctgagattcttttacagttaacttttttataagtaggagt
atactctaaaataatgattaaaagtatagtctagtaaataaatgaaccagtaacatagtcatctattt
ttactatcaagtactatgtaatgtacataattgtatgtgctatacttttacaactggcagctcagtag
gtttgtttacaccagcattgccacaaacacatgagtaatgtgttatgctacaatgtcactagatggta
ggaatttttcagcacacacacacaagtatatatattatatattatattatatattatatatatatatg
tatatatatatatatatagagagagagagagagagagagagagagatggagtcttgctctgtcg
cccaggctggggtgcaatgacacaatctcggctcactgcaacctgactcccaggttcaagtgattctc
ctgcctcagcctcctgagtagctgggactacaggtgcccaccaccatgcccagctaatttttgtattt
ttagtagagacggggtttcaccatgttggccagattggtcccaaactcctgacctcaagtgatccacc
ccactcagcctctcaaagtgctgggattacaggcgtgagccaccgtgcctggccaacaccattataat
cttatgggaccactgtcatacatgtggttcatcattggccaaagcatctgtatttatatatgtatgtt
tcaaattatatatatatatatatatatatatatatatgatagctatacatgaacatacacacacacat
atatagacatatatagcacataaaattggcacatattaagcattttgtaaatatcaaccattacaatt
gttactacttttctcagcaaggctatgaatgctgttccagcctgtcaaaatcacacctgtttaatgtg
ttttacccagcacgaagtcatgtctagttgagtggcttaaaaattgtgatcaaatagctggttagtta
aaaagttatttcactgtgtaaaatacatcccttaaaatgcactgttatttatctcttagttgtagaaa
ttggtttcattttccactatgtttaattgtgactggatcattatagaccctttttttgtagttgttga
ggtttaaagatttaagtttgttatggatgcaagcttttcagttgaccaatgattattagccaatttct
gataaaagaaaaggaaaccgattgccccagggctgctgttttcatttcctcattggaagaagaagcat
agtatagaagaaaggcaaacacaacacattcaacctctgccacc

## Claims

1. Method for *in vitro* or *ex vivo* engineering of a stem cell, comprising introducing into a cell:
a) a gene editing agent comprising, or consisting of:
a.i) a nuclease capable of introducing a double strand break (DSB) in a target site in the cell genome; and
a.ii) a guide RNA (gRNA) targeting the target site;
wherein the target site is located within a functional region of a target gene, wherein the target gene is a gene that is haploinsufficient and/or that is present in single copy in the cell;
and
b) a donor template that comprises:
b.i) a knock-in cassette comprising an exogenous polynucleotide encoding a gene product of interest, or a portion thereof; and
b.ii) homology arms (HAs) on either side of the knock-in cassette, wherein the 5' homology arm has a sequence comprising, or consisting of, a sequence that is homologous to a sequence located 5' of the DSB in the target site of the cell genome and the 3' homology arm has a sequence comprising, or consisting, of a sequence that is homologous to a sequence located 3' of the DSB in the target site of the cell genome;
wherein the donor template comprises an exogenous polynucleotide having sequence capable of reconstituting the target gene upon integration of the knock-in cassette into the target site of the cell by homology-directed repair (HDR) of the DSB,
and wherein integration of the knock-in cassette by HDR of the DSB results in a gene edited cell that expresses the gene product of the target gene and the gene product of interest; preferably wherein the gene product of interest is different from the gene product of the target gene.

2. Kit for *in vitro* or *ex vivo* engineering of a cell, comprising:
a) a gene editing agent comprising, or consisting of:
a.i) a nuclease capable of introducing a double strand break (DSB) in a target site in the cell genome; and
a.ii) a guide RNA (gRNA) targeting the target site;
wherein the target site is a site located within a functional region of a target gene, wherein the target gene is a gene that is haploinsufficient and/or that is present in single copy in the cell; and
b) a donor template that comprises:
b.i) a knock-in cassette comprising an exogenous polynucleotide encoding a gene product of interest, or a portion thereof; and
b.ii) homology arms (HAs) on either side of the knock-in cassette, wherein the 5' homology arm has a sequence comprising or consisting of a sequence that is homologous to a sequence located 5' of the DSB in the target site of the cell genome and the 3' homology arm has a sequence comprising or consisting of a sequence that is homologous to a sequence located 3' of the DSB in the target site of the cell genome;
wherein the donor template comprises an exogenous polynucleotide having sequence capable of reconstituting the target gene, upon integration of the knock-in cassette into the target site of the cell by homology-directed repair (HDR) of the DSB,
such that integration of the knock-in cassette by HDR of the DSB results in a gene edited cell that expresses the gene product of the target gene and the gene product of interest;
the kit optionally comprising c) a cell population;
preferably wherein the gene product of interest is different from the gene product of the target gene.

3. The method of claim 1 or the kit of claim 2, wherein the cell is a hematopoietic stem cells (HSC), a hematopoietic stem and progenitor cells (HSPC), a T cell, a B cell, or a NK cell; preferably a human cell.

4. The method or kit of any one of claims 1-3, wherein the target gene is selected from the group consisting of: *UBA1*, *RPS19*, *ABCB7*, *AIFM1*, *DKC1*,*FLNA*, *HCCS*, *MED12*, *OGT*, *PGK1,* and *UBL4A* genes; preferably the cell being a HSPC.

5. The method or kit of any one of claims 1-4, wherein the target site is within an exon of *UBA1* gene, *RPS19* gene or *OGT* gene*.*

6. The method or kit of any one of claims 1-5, wherein the gRNA of the gene editing agent targets a target site having sequence comprising, or consisting of, any one of sequences SEQ ID NO: 1, 3, 5 or 27.

7. The method or kit of any one of claims 1-6, wherein the gRNA of the gene editing agent targets a target site having sequence comprising, or consisting of SEQ ID NO: 5.

8. The method or kit of claim 7, wherein the knock-in cassette of the donor template comprises in sequence from the 5' end to the 3' end:
- an exogenous polynucleotide having sequence capable of reconstituting *UBA1* said sequence being SEQ ID NO: 36,
- an exogenous polynucleotide encoding for a gene product of interest, under the control of an exogenous promoter, preferably of a promoter having sequence SEQ ID NO: 37, 55 or 56, and
- a polyadenylation signal, preferably a bGH polyadenylation signal having sequence SEQ ID NO: 39;
preferably wherein the left and right HAs of the donor template have sequences comprising, or consisting of, SEQ ID NO: 40 and 41, respectively.

9. The method or kit of any one of claims 1-5, wherein the gRNA of the gene editing agent targets a target site having sequence comprising, or consisting of, SEQ ID NO: 1 .

10. The method or kit of claim 9, wherein the knock-in cassette of the donor template comprises in sequence from the 5' end to the 3' end:
- an exogenous polynucleotide having sequence capable of reconstituting *RPS19* gene said sequence being sequence SEQ ID NO: 42,
- a P2A domain having sequence SEQ ID NO: 43,
- an exogenous polynucleotide encoding for a gene product of interest, and
- a polyadenylation signal, preferably a bGH polyadenylation signal having sequence SEQ ID NO: 39;
preferably wherein the left and right HAs of the donor template have sequences comprising, or consisting of, SEQ ID NO: 44 and 45, respectively.

11. The method or kit of any one of claims 1-5, wherein the gRNA of the gene editing agent targets a target site having sequence comprising, or consisting of, SEQ ID NO: 27.

12. The method or kit of claim 11, wherein the knock-in cassette of the donor template comprises in sequence from the 5' end to the 3' end:
- an exogenous polynucleotide having sequence capable of reconstituting *OGT* gene said sequence being SEQ ID NO: 49,
- an exogenous polynucleotide encoding for a gene product of interest, under the control of an exogenous promoter, preferably of a promoter having sequence SEQ ID NO: 37, 55 or 56, and
- a polyadenylation signal, preferably a bGH polyadenylation signal having sequence SEQ ID NO: 39;
preferably wherein the left and right HAs of the donor template have sequences comprising, or consisting of, SEQ ID NO: 50 and 51, respectively.

13. A population of gene edited cells obtained by the method of any one of claims 1-12

14. A pharmaceutical composition comprising the kit of any one of claims 2-12, or the population of cells of claim 13, and suitable pharmaceutically acceptable excipients.

15. The kit of any one of claims 2-12, or the population of cells of claim 13, or the pharmaceutical formulation of claim 14, for use as medicament, preferably for use as medicament in gene therapy.
